(19) Europäisches Patentamt
European Patent Office
Office européen des brevets

(11) **EP 4 327 852 B1**

(12) **EUROPEAN PATENT SPECIFICATION**

(45) Date of publication and mention
of the grant of the patent:
**15.10.2025 Bulletin 2025/42**

(21) Application number: **23193150.2**

(22) Date of filing: **24.08.2023**

(51) International Patent Classification (IPC):
**A61M 16/06** $^{(2006.01)}$

(52) Cooperative Patent Classification (CPC):
(C-Sets available)
**A61M 16/06; A61M 16/202; A61M 16/208;**
A61M 16/0683; A61M 2016/0027; A61M 2016/0033;
A61M 2202/0225; A61M 2205/3365; A61M 2205/42;
A61M 2205/505; A61M 2230/432          (Cont.)

(54) **APPARATUS FOR INCREASING BREATHING COMFORT DURING RESPIRATORY THERAPY**

VORRICHTUNG ZUR ERHÖHUNG DES ATEMKOMFORTS WÄHREND EINER ATEMTHERAPIE

APPAREIL POUR AUGMENTER LE CONFORT RESPIRATOIRE PENDANT UNE THÉRAPIE RESPIRATOIRE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **24.08.2022 AU 2022902417**

(43) Date of publication of application:
**28.02.2024 Bulletin 2024/09**

(73) Proprietor: **ResMed Pty Ltd
Bella Vista, NSW 2153 (AU)**

(72) Inventors:
• **Dent, Michael James**
**Bella Vista, NSW (AU)**
• **Lee Teh, Ting**
**Bella Vista, NSW (AU)**

(74) Representative: **Vossius & Partner
Patentanwälte Rechtsanwälte mbB
Siebertstraße 3
81675 München (DE)**

(56) References cited:
**WO-A1-2017/049357     US-A1- 2014 246 024**

(52) Cooperative Patent Classification (CPC): (Cont.)

C-Sets
A61M 2202/0225, A61M 2202/0085

**Description**

1 BACKGROUND OF THE TECHNOLOGY

1.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use. The invention relates to a patient interface.

1.2 DESCRIPTION OF THE RELATED ART

**1.2.1 Human Respiratory System and its Disorders**

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"Respiratory Physiology"*, by John B. West, Lippincott Williams & Wilkins, 9th edition published 2012.

**1.2.2 Therapies**

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

**1.2.2.1 Respiratory pressure therapies**

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Some respiratory therapy patients may dislike the experience of breathing pressurised air and may have difficulty falling asleep while wearing a patient interface which is being supplied with air at full treatment pressure (or even at a reduced pressure). Difficulty in falling asleep may be a factor in reduced compliance in a significant proportion of patients.

In an attempt to mitigate this problem, some RPT devices of the prior art may supply air at a pressure lower than treatment pressure for a period of time before increasing the supplied pressure to the treatment pressure. In some prior art examples, the pressure may be increased after a predetermined length of time has passed. However, in some other prior art examples the RPT device may increase the supplied pressure after the RPT device has determined that the patient has fallen asleep. Sleep detection may rely on pressure changes, caused by the patient's breathing, being transmitted down the air circuit and detected by sensors in the RPT.

Although such RPT devices do go some way towards addressing the problem of patients having difficulty falling asleep while wearing a patient interface which is being supplied with air at full treatment pressure, the need for adequate $CO_2$ washout of the patient interface puts an effective lower limit on the pressure which can be supplied to the patient interfaces of the prior art. The pressure at this lower limit may still be sufficiently high to cause discomfort and/or difficulty in falling asleep for some patients.

Patients may be sensitive to sudden changes in pressure and/or to noises or vibrations from the therapy system, and so these should be avoided in order to avoid rousing the patient.

### 1.2.3 Respiratory Therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

### 1.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 $cmH_2O$ relative to ambient pressure. For other forms of therapy, such as the delivery of oxygen, the patient interface may not include a seal sufficient to facilitate delivery to the airways of a supply of gas at a positive pressure of about 10 $cmH_2O$. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

#### *1.2.3.1.1 Seal-forming structure*

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFTTM nasal pillows mask, SWIFTTM II nasal pillows mask, SWIFTTM LT nasal pillows mask, SWIFTTM FX nasal pillows mask and MIRAGE LIBERTYTM full-face mask. The following patent applications, assigned to ResMed Limited, describe examples of nasal pillows masks: International Patent Application WO2004/073,778 (describing amongst other things aspects of the ResMed Limited SWIFTTM nasal pillows), US Patent Application 2009/0044808 (describing amongst other things aspects of the ResMed Limited SWIFTTM LT nasal pillows); International Patent Applications WO 2005/063,328 and WO 2006/130,903 (describing amongst other things aspects of the ResMed Limited MIRAGE LIBERTYTM full-face mask); International Patent Application WO 2009/052,560 (describing amongst other things aspects of the ResMed Limited SWIFTTM FX nasal pillows).

### *1.2.3.1.2 Positioning and stabilising*

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 1.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

One known RPT device used for treating sleep disordered breathing is the S9 Sleep Therapy System, manufactured by ResMed Limited. Another example of an RPT device is a ventilator. Ventilators such as the ResMed Stellar™ Series of Adult and Paediatric Ventilators may provide support for invasive and non-invasive nondependent ventilation for a range of patients for treating a number of conditions such as but not limited to NMD, OHS and COPD.

The ResMed Elisée™ 150 ventilator and ResMed VS III™ ventilator may provide support for invasive and non-invasive dependent ventilation suitable for adult or paediatric patients for treating a number of conditions. These ventilators provide volumetric and barometric ventilation modes with a single or double limb circuit. RPT devices typically comprise a pressure generator, such as a motor-driven blower or a compressed gas reservoir, and are configured to supply a flow of air to the airway of a patient. In some cases, the flow of air may be supplied to the airway of the patient at positive pressure. The outlet of the RPT device is connected via an air circuit to a patient interface such as those described above.

### 1.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 1.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition, in cooler climates, warm air applied generally to the face area in and about the patient interface is more comfortable than cold air.

### 1.2.3.5 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system.

Existing processes to communicate and manage such data can be one or more of costly, time-consuming, and error-prone.

### 1.2.3.6 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

Table of noise of prior masks (ISO 17510-2:2007, 10 cmH2O pressure at 1m)

| Mask name | Mask type | A-weighted sound power level dB(A) (uncertainty) | A-weighted sound pressure dB(A) (uncertainty) | Year (approx.) |
|---|---|---|---|---|
| Glue-on (*) | nasal | 50.9 | 42.9 | 1981 |

(continued)

| Mask name | Mask type | A-weighted sound power level dB(A) (uncertainty) | A-weighted sound pressure dB(A) (uncertainty) | Year (approx.) |
|---|---|---|---|---|
| ResCare standard (*) | nasal | 31.5 | 23.5 | 1993 |
| ResMed MirageTM (*) | nasal | 29.5 | 21.5 | 1998 |
| ResMed UltraMirageT M | nasal | 36 (3) | 28 (3) | 2000 |
| ResMed Mirage ActivaTM | nasal | 32 (3) | 24 (3) | 2002 |
| ResMed Mirage MicroTM | nasal | 30 (3) | 22 (3) | 2008 |
| ResMed MirageTM SoftGel | nasal | 29 (3) | 22 (3) | 2008 |
| ResMed MirageTM FX | nasal | 26 (3) | 18 (3) | 2010 |
| ResMed Mirage SwiftTM (*) | nasal pillows | 37 | 29 | 2004 |
| ResMed Mirage SwiftTM II | nasal pillows | 28 (3) | 20 (3) | 2005 |
| ResMed Mirage SwiftTM LT | nasal pillows | 25 (3) | 17 (3) | 2008 |
| ResMed AirFit P10 | nasal pillows | 21 (3) | 13 (3) | 2014 |
| (* one specimen only, measured using test method specified in ISO 3744 in CPAP mode at 10 cmH2O) | | | | |

### 1.2.4 Screening, Diagnosis, and Monitoring Systems

Polysomnography (PSG) is a conventional system for diagnosis and monitoring of cardio-pulmonary disorders, and typically involves expert clinical staff to apply the system. PSG typically involves the placement of 15 to 20 contact sensors on a patient in order to record various bodily signals such as electroencephalography (EEG), electrocardiography (ECG), electrooculograpy (EOG), electromyography (EMG), etc. PSG for sleep disordered breathing has involved two nights of observation of a patient in a clinic, one night of pure diagnosis and a second night of titration of treatment parameters by a clinician. PSG is therefore expensive and inconvenient. In particular, it is unsuitable for home screening / diagnosis / monitoring of sleep disordered breathing.

Screening and diagnosis generally describe the identification of a condition from its signs and symptoms. Screening typically gives a true / false result indicating whether or not a patient's SDB is severe enough to warrant further investigation, while diagnosis may result in clinically actionable information. Screening and diagnosis tend to be one-off processes, whereas monitoring the progress of a condition can continue indefinitely. Some screening / diagnosis systems are suitable only for screening / diagnosis, whereas some may also be used for monitoring.

Clinical experts may be able to screen, diagnose, or monitor patients adequately based on visual observation of PSG signals. However, there are circumstances where a clinical expert may not be available, or a clinical expert may not be affordable. Different clinical experts may disagree on a patient's condition. In addition, a given clinical expert may apply a different standard at different times.

US 2014/246024 AI relates to a device for treating a patient suffering from obstructive sleep apnea or snoring can include an expiratory valve connected to a manifold. The expiratory valve can include a body portion including a feedback port configured to be connected to an air flow generator. The expiratory valve can include a plunger at least partially disposed in the body portion. The expiratory valve can include a pressurizing chamber positioned between an end of the plunger and an end of the expiratory valve. The pressurizing chamber can be configured to receive air from the air flow generator through the feedback port.

WO 2017/049357 AI relates to an elbow component that houses a pair of anti-asphyxia valves.

2 BRIEF SUMMARY OF THE TECHNOLOGY

The invention is set out in the appended set of claims.

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

## 2.1 BREATHE FREE VALVES

One form of the present technology comprises a patient interface comprising a plenum chamber and a valve, or a plurality of valves, wherein the or each valve is in fluid communication with the plenum chamber, the or each valve comprising a valve member and a valve seat portion, wherein the or each valve member is biased away from the seat portion and is configured to close when a pressure within the plenum chamber exceeds a predetermined valve closing pressure..

In examples:

a) only one valve is provided;

b) the valve member comprises a plurality of apertures configured to form a vent when the valve member is closed;

c) the valve member is configured to close when a pressure within the plenum chamber exceeds 1 cmH2O;

d) the valve member is configured to close when a pressure within the plenum chamber exceeds 0.5 cmH2O;

e) the valve member may be configured to reduce the area available for flow through the valve in proportion to an increase in pressure in the plenum chamber over a plenum chamber pressure range of 0 cmH2O to 4 cmH2O;

f) the valve member comprises a plurality of flap sections, each flap section connected to an adjacent flap section by a hinge portion;

g) a plurality of the valves are provided;

h) the predetermined valve closing pressure is different for one of the valves than it is for another of the valves;

i) the predetermined valve closing pressure for a first of the valves is 0.5 cmH2O and the predetermined valve closing pressure for a second of the valves is 1 cmH2O.

j) the valve seat portion is constructed from a soft material;

k) the valve seat portion is constructed from a material having a Shore A Durometer hardness of less than 40, preferably between 10-40;

l) the valve seat material is constructed from foam;

m) the valve has a flow area of at least 330 mm2 or the plurality of valves have an aggregate flow area of at least 330mm2.

n) at least one of the valves is an electrically actuated valve;

o) the patient interface comprises a sensor configured to sense a characteristic of the air within the plenum chamber, wherein the characteristic is indicative of whether a patient wearing the patient interface is asleep; and/or

p) the patient interface comprises a sensor configured to sense an orientation of the patient interface to determine, in use, whether a patient wearing the patient interface is lying on their back or on their side.

## 2.2 SLEEP DETECTION AT LOW PRESSURE

One form of the present technology comprises a method of detecting when a patient has fallen asleep while wearing a patient interface which is supplied with breathable gas such that a pressure within a plenum chamber of the patient interface is less than 2 cmH2O,
the method comprising receiving information related to the patient's breathing from one or more of: a flow sensor; a pressure sensor; a CO2 sensor and a volatile organic compounds (VOC) sensor, wherein the or each sensor is positioned within or immediately adjacent the plenum chamber, the method further comprising determining whether the patient is asleep based on the information.

Another form of the present technology comprises a method of detecting when a patient has fallen asleep while wearing a patient interface which is supplied with breathable gas such that a pressure within a plenum chamber of the patient interface is less than 2 cmH2O,

the method comprising receiving information related to the patient's movement wirelessly from a device which is worn by the patient, or which is in contact with the patient's bed, wherein the device comprises at least one accelerometer, the method further comprising determining whether the patient is asleep based on the information.

## 2.3 CESSATION OF THERAPY PRESSURE

Another form of the present technology comprises a method of providing respiratory pressure therapy to a patient, the method comprising:

- providing breathable gas at a therapy pressure while the patient is asleep;
- detecting when the patient has woken up, or determining that the patient is likely to wake up within a predetermined period in the future; and
- when the patient wakes up, or within the predetermined period, reducing the pressure of the breathable gas supplied to the patient to below therapy pressure.

In examples:

a) the method comprises the step of supplying the breathable gas at below therapy pressure until the patient falls asleep; and/or
b) the predetermined period is less than 12 minutes.

Another form of the present technology comprises a method of providing respiratory pressure therapy to a patient, the method comprising monitoring a sleeping position of the patient and:

- if the patient is lying in a position in which snoring or OSA is likely, supplying breathable gas at a therapy pressure; and
- if the patient is lying in a position in which snoring or OSA is unlikely, supplying breathable gas at less than the therapy pressure.

In examples:

a) the method comprises receiving information regarding the sleeping position from a device worn by the user or another sleep position monitoring device;
b) the method is performed by an RPT device.

## 2.4 SEAL CHECK

Another form of the present technology comprises a method of providing respiratory pressure therapy to a patient, wherein the patient is wearing a patient interface, the method comprising:

- Supplying breathable gas to the patient interface at an initial pressure which is less than a predetermined therapy pressure;
- increasing the pressure of the breathable gas supplied to the patient interface to the predetermined therapy pressure over a period of no more than 2 minutes;
- checking for leaks from the patient interface during the increasing pressure step; and
- if no unacceptable leak is detected during the increasing pressure step, reducing pressure to below 0.2 cmH2O until the patient falls asleep or a predetermined time has passed;
- if an unacceptable leak is detected during the increasing pressure step, pausing the increase in pressure and prompting the patient to adjust the patient interface, then resuming the increasing pressure step.

In examples:

a) the initial pressure is 4 cmH2O or less;

b) the initial pressure is 1 cmH2O or less; and/or

c) the step of increasing the pressure of the breathable gas supplied to the patient interface to a therapy pressure occurs over a period of no more than 1 minute.

Another aspect of one form of the present technology is a patient interface that is moulded or otherwise constructed with a perimeter shape which is complementary to that of an intended wearer.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

## 3 BRIEF DESCRIPTION OF THE DRAWINGS

The present technology is illustrated by way of example, and not by way of limitation, in the figures of the accompanying drawings, in which like reference numerals refer to similar elements including:

### 3.1 RESPIRATORY THERAPY SYSTEMS

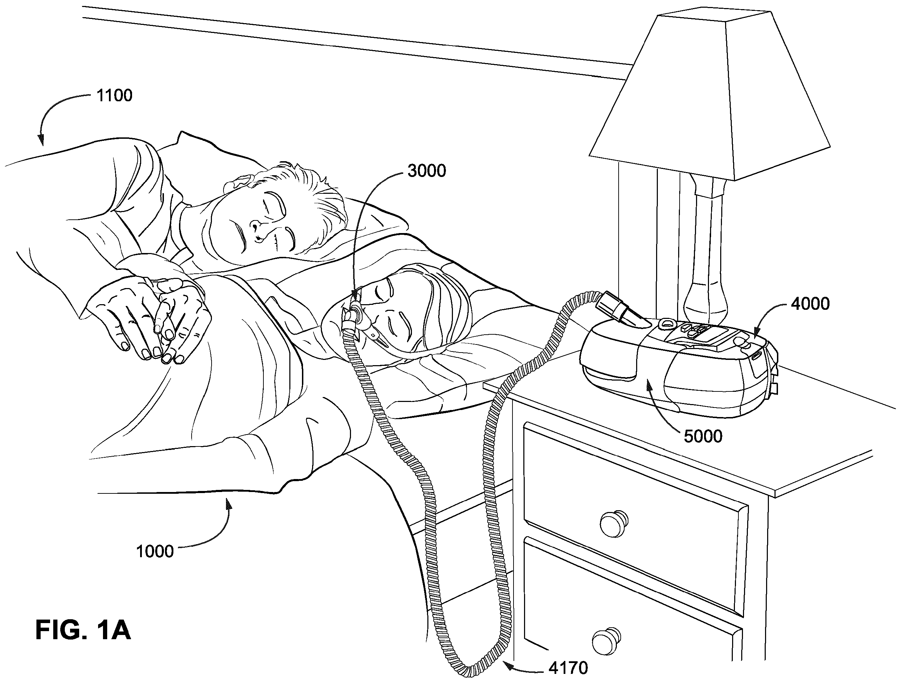

Fig. 1A shows a system including a patient 1000 wearing a patient interface 3000, in the form of nasal pillows, receiving a supply of air at positive pressure from an RPT device 4000. Air from the RPT device 4000 is humidified in a humidifier 5000, and passes along an air circuit 4170 to the patient 1000. A bed partner 1100 is also shown. The patient is sleeping in a supine sleeping position.

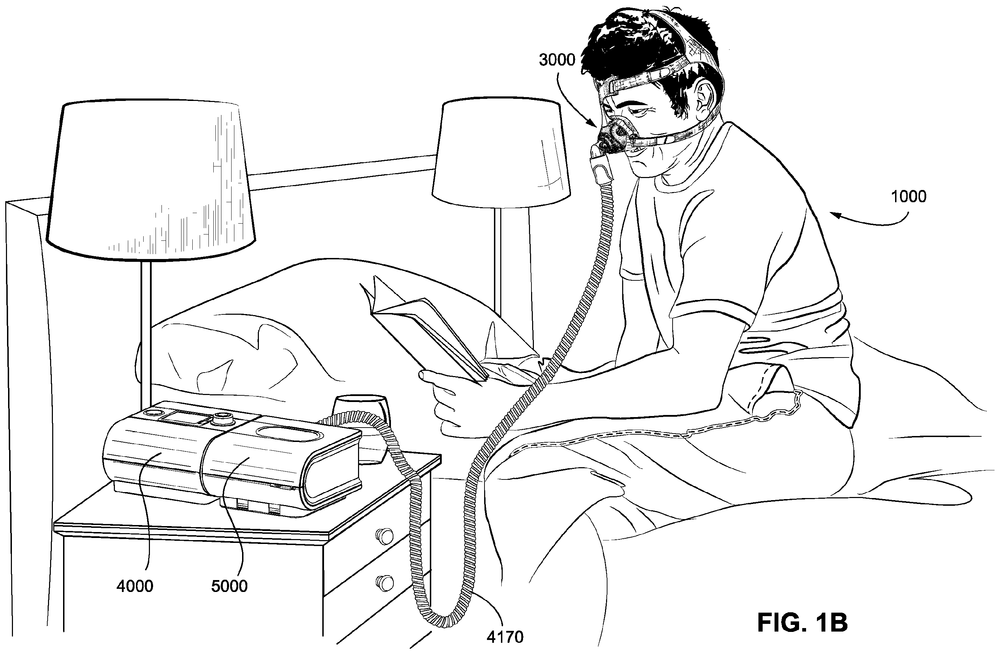

Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

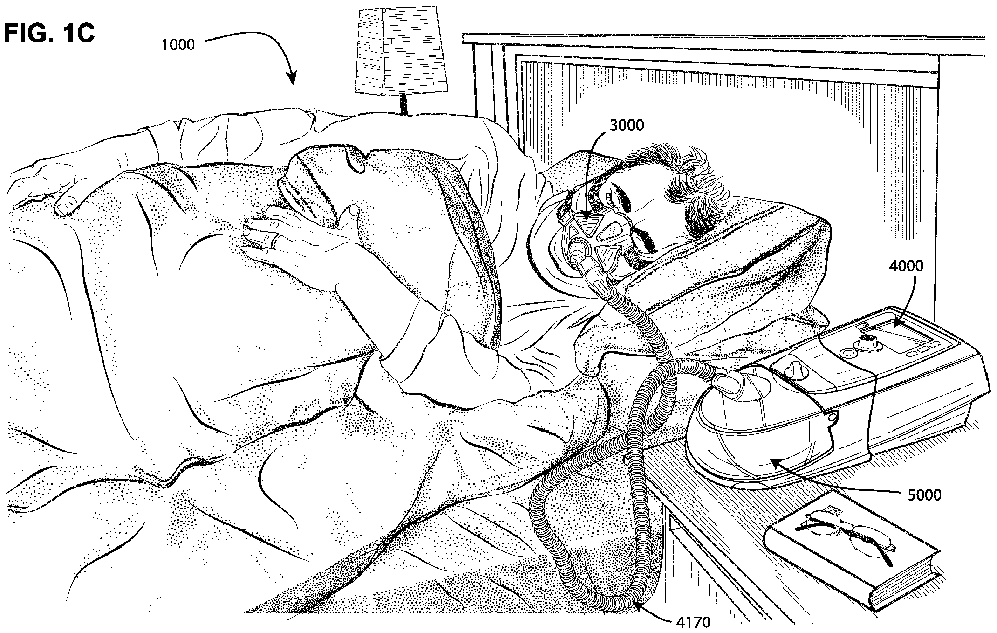

Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 3.2 RESPIRATORY SYSTEM AND FACIAL ANATOMY

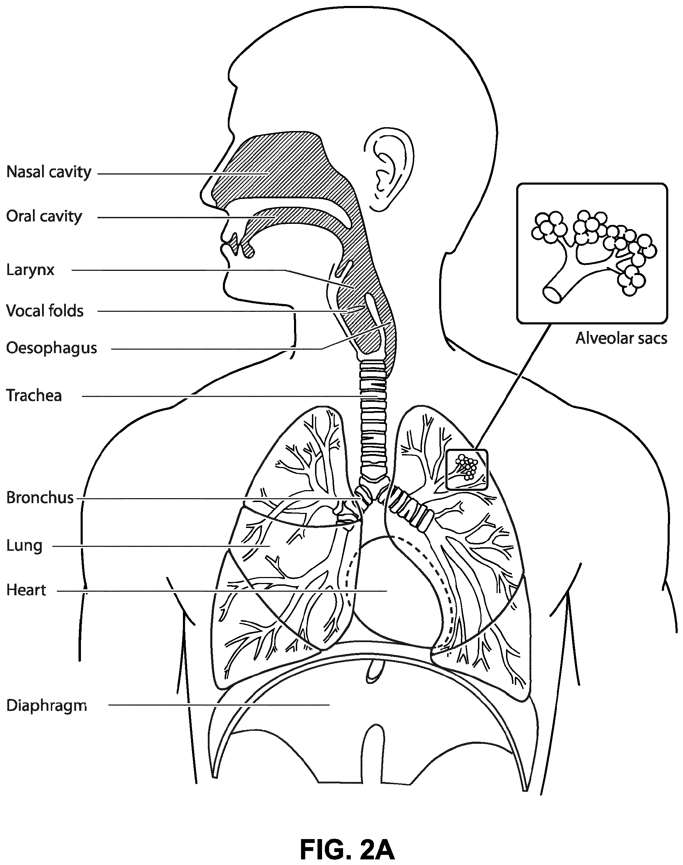

Fig. 2A shows an overview of a human respiratory system including the nasal and oral cavities, the larynx, vocal folds, oesophagus, trachea, bronchus, lung, alveolar sacs, heart and diaphragm.

### 3.3 PATIENT INTERFACE

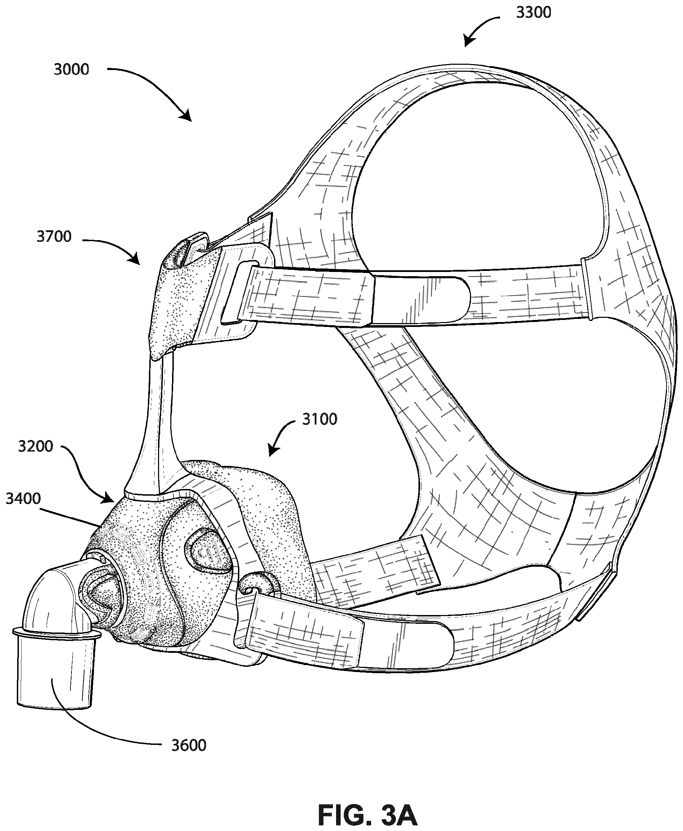

Fig. 3A shows a patient interface in the form of a nasal mask in accordance with one form of the present technology.

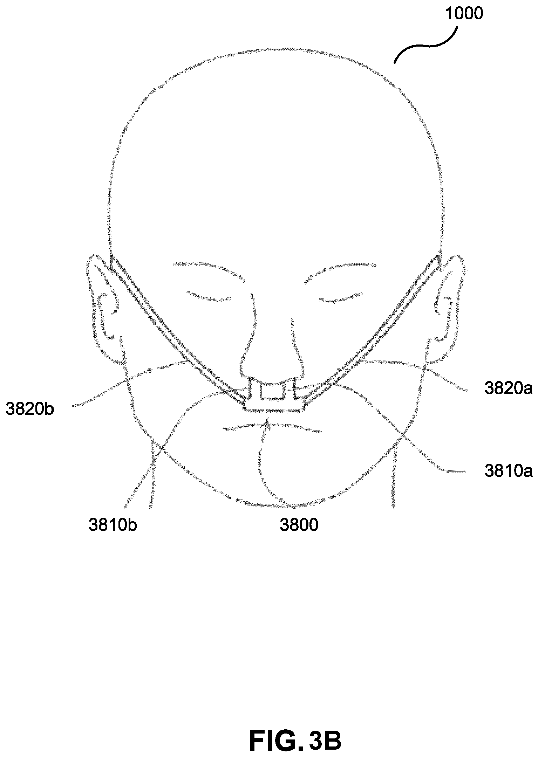

Fig. 3B shows a patient interface in the form of a nasal cannula in accordance with one form of the present technology.

### 3.4 RPT DEVICE

Fig. 4A shows an RPT device in accordance with one form of the present technology.

Fig. 4B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

Fig. 4C is a schematic diagram of the electrical components of an RPT device in accordance with one form of the present technology.

Fig. 4D is a schematic diagram of the algorithms implemented in an RPT device in accordance with one form of the present technology.

Fig. 4E is a flow chart illustrating a method carried out by the therapy engine module of Fig. 4D in accordance with one form of the present technology.

### 3.5 HUMIDIFIER

Fig. 5A shows an isometric view of a humidifier in accordance with one form of the present technology.

Fig. 5B shows an isometric view of a humidifier in accordance with one form of the present technology, showing a humidifier reservoir 5110 removed from the humidifier reservoir dock 5130.

### 3.6 BREATHING WAVEFORMS
Fig. 6 shows a model typical breath waveform of a person while sleeping.

### 3.7 APPARATUS OF THE PRESENT TECHNOLOGY

Fig. 7A shows a perspective view of a patient interface of one form of the technology with an open valve.

Fig. 7B shows the patient interface of Fig. 7A with the valve closed.

Fig. 8A shows a perspective view of a patient interface of another form of the technology with four open valves.

Fig. 8B shows the patient interface of Fig. 8A with the valves closed.

Fig. 9A shows a perspective view of a patient interface of another form of the technology with an open valve.

Fig. 9B shows a side view of the valve member of the interface of Fig. 9A in various states of closure.

Fig. 9C shows the patient interface of Fig. 9A with the valve fully closed.

Fig. 10 shows a patient interface according to another form of the technology with a portion cut away to show a sensor.

Fig. 11 shows a patient 1000 wearing a patient interface 3000 and a wearable device 3900. A smart phone 3910 and sensor 3920 are also shown.

### 4 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.
The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

## 4.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

Various methods and apparatus described herein may be configured to be used with a method of providing therapy in which the pressure supplied to the patient interface is maintained at a very low level (e.g. less than 1 cmH2O or less than 0.5 cmH2O, or even less than 0.2 cmH2O) until the patient is asleep. In examples, once the patient has fallen asleep, the pressure within the patient interface is increased relatively slowly (e.g. 1, 2 or even 3 minutes per cmH2O increase) until therapy pressure is reached (e.g. until a pressure of at least 4 cmH2O is reached). Such a method may be referred to herein as "Breathe Free" therapy.

During this low-pressure period it is important that flow rates to the patient interface stay sufficiently high to provide adequate CO2 washout and to avoid excess CO2 rebreathing (e.g. such that the patient is subject to less than around 12.5% rebreathed CO2).

## 4.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000 or 3800.

## 4.3 PATIENT INTERFACE

A non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

An unsealed patient interface 3800, in the form of a nasal cannula, includes nasal prongs 3810a, 3810b which can deliver air to respective nares of the patient 1000 via respective orifices in their tips. Such nasal prongs do not generally form a seal with the inner or outer skin surface of the nares. This type of interface results in one or more gaps that are present in use by design (intentional) but they are typically not fixed in size such that they may vary unpredictably by movement during use. This can present a complex pneumatic variable for a respiratory therapy system when pneumatic control and/or assessment is implemented, unlike other types of mask-based respiratory therapy systems. The air to the nasal prongs may be delivered by one or more air supply lumens 3820a, 3820b that are coupled with the nasal cannula-type unsealed patient interface 3800. The lumens 3820a, 3820b lead from the nasal cannula-type unsealed patient interface 3800 to a respiratory therapy device via an air circuit. The unsealed patient interface 3800 is particularly suitable for delivery of flow therapies, in which the RPT device generates the flow of air at controlled flow rates rather than controlled pressures. The "vent" or gap at the unsealed patient interface 3800, through which excess airflow escapes to ambient, is the passage between the end of the prongs 3810a and 3810b of the nasal cannula-type unsealed patient interface 3800 via the patient's nares to atmosphere.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 cmH2O with respect to ambient.

The patient interface 3000 in accordance with one form of the present technology is constructed and arranged to be able to provide a supply of air at a positive pressure of at least 10 cmH2O with respect to ambient.

The patient interface 3000 in accordance with one form of the present technology is constructed and arranged to be able to provide a supply of air at a positive pressure of at least 20 cmH2O with respect to ambient.

### 4.3.1 Seal-forming structure

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs- the actual sealing surface- may change within a given treatment session, from day to day, and from patient to patient, depending on a range of factors including for example, where the patient interface was placed on the face, tension in the positioning and stabilising structure and the shape of a patient's face.

In one form the target seal-forming region is located on an outside surface of the seal-forming structure 3100.

In certain forms of the present technology, the seal-forming structure 3100 is constructed from a biocompatible material, e.g. silicone rubber.

A seal-forming structure 3100 in accordance with the present technology may be constructed from a soft, flexible, resilient material such as silicone.

In certain forms of the present technology, a system is provided comprising more than one a seal-forming structure 3100, each being configured to correspond to a different size and/or shape range. For example the system may comprise one form of a seal-forming structure 3100 suitable for a large sized head, but not a small sized head and another suitable for a small sized head, but not a large sized head.

### 4.3.1.1 Sealing mechanisms

In one form, the seal-forming structure includes a sealing flange utilizing a pressure assisted sealing mechanism. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. Support flange may be relatively thicker than the sealing flange. The support flange is disposed between the sealing flange and the marginal edge of the plenum chamber 3200, and extends at least part of the way around the perimeter. The support flange is or includes a springlike element and functions to support the sealing flange from buckling in use.

In one form, the seal-forming structure may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure comprises a tension portion. In use, the tension portion is held in tension, e.g. by adjacent regions of the sealing flange.

In one form, the seal-forming structure comprises a region having a tacky or adhesive surface.

In certain forms of the present technology, a seal-forming structure may comprise one or more of a pressure-assisted sealing flange, a compression sealing portion, a gasket sealing portion, a tension portion, and a portion having a tacky or adhesive surface.

### 4.3.1.2 Nose bridge or nose ridge region

In one form, the non-invasive patient interface 3000 comprises a seal-forming structure that forms a seal in use on a nose bridge region or on a nose-ridge region of the patient's face.

In one form, the seal-forming structure includes a saddle-shaped region constructed to form a seal in use on a nose bridge region or on a nose-ridge region of the patient's face.

### 4.3.1.3 Upper lip region

In one form, the non-invasive patient interface 3000 comprises a seal-forming structure that forms a seal in use on an upper lip region (that is, the lip superior) of the patient's face.

In one form, the seal-forming structure includes a saddle-shaped region constructed to form a seal in use on an upper lip region of the patient's face.

### 4.3.1.4 Chin-region

In one form the non-invasive patient interface 3000 comprises a seal-forming structure that forms a seal in use on a chin-region of the patient's face.

In one form, the seal-forming structure includes a saddle-shaped region constructed to form a seal in use on a chin-region of the patient's face.

### 4.3.1.5 Forehead region

In one form, the seal-forming structure that forms a seal in use on a forehead region of the patient's face. In such a form, the plenum chamber may cover the eyes in use.

### 4.3.1.6 Nasal pillows

In one form the seal-forming structure of the non-invasive patient interface 3000 comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

### 4.3.2 Plenum chamber

The plenum chamber 3200 has a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200. In some forms, the plenum chamber 3200 and the seal-forming structure 3100 are formed from a single homogeneous piece of material.

In certain forms of the present technology, the plenum chamber 3200 does not cover the eyes of the patient in use. In other words, the eyes are outside the pressurised volume defined by the plenum chamber. Such forms tend to be less obtrusive and / or more comfortable for the wearer, which can improve compliance with therapy.

In certain forms of the present technology, the plenum chamber 3200 is constructed from a transparent material, e.g. a transparent polycarbonate. The use of a transparent material can reduce the obtrusiveness of the patient interface, and help improve compliance with therapy. The use of a transparent material can aid a clinician to observe how the patient interface is located and functioning.

In certain forms of the present technology, the plenum chamber 3200 is constructed from a translucent material. The use of a translucent material can reduce the obtrusiveness of the patient interface, and help improve compliance with therapy.

### 4.3.3 Positioning and stabilising structure

The seal-forming structure 3100 of the patient interface 3000 of the present technology may be held in sealing position in use by the positioning and stabilising structure 3300.

In one form the positioning and stabilising structure 3300 provides a retention force at least sufficient to overcome the effect of the positive pressure in the plenum chamber 3200 to lift off the face.

In one form the positioning and stabilising structure 3300 provides a retention force to overcome the effect of the gravitational force on the patient interface 3000.

In one form the positioning and stabilising structure 3300 provides a retention force as a safety margin to overcome the potential effect of disrupting forces on the patient interface 3000, such as from tube drag, or accidental interference with the patient interface.

In one form of the present technology, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

In one form of the present technology, a positioning and stabilising structure 3300 is provided that is configured so as not to be too large and bulky to prevent the patient from lying in a supine sleeping position with a back region of the patient's head on a pillow.

In one form of the present technology, a positioning and stabilising structure 3300 is provided that is configured so as not to be too large and bulky to prevent the patient from lying in a side sleeping position with a side region of the patient's head on a pillow.

In one form of the present technology, a positioning and stabilising structure 3300 is provided with a decoupling portion located between an anterior portion of the positioning and stabilising structure 3300, and a posterior portion of the positioning and stabilising structure 3300. The decoupling portion does not resist compression and may be, e.g. a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, a positioning and stabilising structure 3300 comprises a strap constructed from a laminate of a fabric patientcontacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the present technology, a positioning and stabilising structure 3300 comprises a strap that is extensible, e.g. resiliently extensible. For example the strap may be configured in use to be in tension, and to direct a force to draw a seal-forming structure into sealing contact with a portion of a patient's face. In an example the strap may be configured as a tie.

In one form of the present technology, the positioning and stabilising structure comprises a first tie, the first tie being constructed and arranged so that in use at least a portion of an inferior edge thereof passes superior to an otobasion superior of the patient's head and overlays a portion of a parietal bone without overlaying the occipital bone.

In one form of the present technology suitable for a nasal-only mask or for a full-face mask, the positioning and stabilising structure includes a second tie, the second tie being constructed and arranged so that in use at least a portion of a superior edge thereof passes inferior to an otobasion inferior of the patient's head and overlays or lies inferior to the occipital bone of the patient's head.

In one form of the present technology suitable for a nasal-only mask or for a full-face mask, the positioning and stabilising structure includes a third tie that is constructed and arranged to interconnect the first tie and the second tie to reduce a tendency of the first tie and the second tie to move apart from one another.

In certain forms of the present technology, a positioning and stabilising structure 3300 comprises a strap that is bendable and e.g. non-rigid. An advantage of this aspect is that the strap is more comfortable for a patient to lie upon while the patient is sleeping.

In certain forms of the present technology, a positioning and stabilising structure 3300 comprises a strap constructed to be breathable to allow moisture vapour to be transmitted through the strap,

In certain forms of the present technology, a system is provided comprising more than one positioning and stabilizing structure 3300, each being configured to provide a retaining force to correspond to a different size and/or shape range. For example the system may comprise one form of positioning and stabilizing structure 3300 suitable for a large sized head, but not a small sized head, and another. Suitable for a small sized head, but not a large sized head.

### 4.3.4 Vent

In one form, the patient interface 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled $CO_2$ by the patient while maintaining the therapeutic pressure in the plenum chamber in use.

One form of vent 3400 in accordance with the present technology comprises a plurality of holes, for example, about 20 to about 80 holes, or about 40 to about 60 holes, or about 45 to about 55 holes.

The vent 3400 may be located in the plenum chamber 3200. Alternatively, the vent 3400 may be located in a decoupling structure, e.g., a swivel. In examples, more than one vent may be provided.

### 4.3.5 Breathe free valve

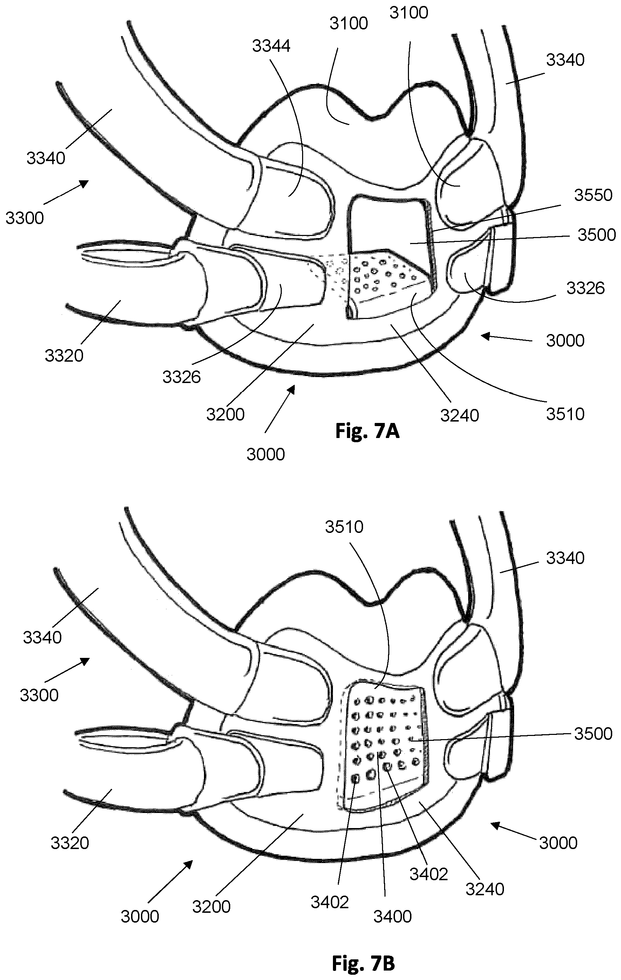

Each of the examples described below with reference to Figs. 7A to 9C, may be configured for use with the "Breathe Free" therapy method described above.

The patient interfaces shown in Figs. 7A-9C may be substantially similar to those described in PCT publication WO2019/183680, the contents of which is included herein by reference (in particular the examples described with reference to Figs. 184 and 185). However, the present technology may be applied to other designs of patient interface 3000, including other designs with alternative positioning and stabilising structures (e.g. positioning and stabilising structures which do not comprise conduits).

The patient interfaces 3000 shown in Figs. 7A to 9C comprise a positioning and stabilising structure 3300 to hold the plenum chamber 3200 in sealing position on the patient's face in use. The positioning and stabilising structure 3300 in this example comprises a pair of headgear tubes 3340. The pair of headgear tubes 3340 are connected to each other at their superior ends and are each configured to lie against superior and lateral surfaces of the patient's head in use. Each of the headgear tubes 3340 may be configured to lie between and eye and an ear of the patient in use. The inferior end of each headgear tube 3340 is configured to fluidly connect to the plenum chamber 3200. In this example, the inferior end of each headgear tube 3340 connects to a headgear tube connector 3344 configured to connect to the shell 3240 of the plenum chamber 3200. The positioning and stabilising structure 3300 comprises a conduit headgear inlet (not shown) at the junction of the two headgear tubes 3340. The conduit headgear inlet is configured to receive a pressurised flow of gas, for example via an elbow comprising a connection port 3600, and allow the flow of gas into hollow interiors of the headgear tubes 3340. The headgear tubes 3340 supply the pressurised flow of gas to the plenum chamber 3200.

In the example shown, the positioning and stabilising structure also comprises a pair of straps 3320 which extend around the back of the patient's head. The straps 3320 comprises a strap clip 3326 provided to the anterior end of each of the straps 3320. Each of the strap clips 3326 is configured to connect to a lower connection point on the plenum chamber 3200.

Referring next to Figs. 7A and 7B, a patient interface is shown with a valve 3500 provided to an anterior surface thereof, e.g. in a shell 3240 of the patient interface. The valve 3500 may have a relatively large flow area (in one example, about 330 mm2) when fully open, such that the pressure in the plenum chamber 3200 can be maintained at a very low level, e.g. less than 1 cmH2O, more preferably less than 0.5cmH2O, more preferably less than 0.2cmH2O, even when the flow rate to the patient interface is sufficiently high to ensure there is adequate $CO_2$ washout (e.g. less than around 12.5% rebreathed $CO_2$). In examples, the flow rate to the patient interface at these low pressures may be between around 10 1/min and around 15 l/min. The flow area required may be highly dependent on the geometry of the particular patient interface 3000 which the valves 3500 are used with. The volume of deadspace within the patient interface 3000 may be one important factor. The positioning of the valve(s) 3500 may also affect the degree of $CO_2$ washout provided for a given valve area. In examples, any AAVs provided to the patient interface may also assist with washout at very low pressures (e.g less than 0.2 cmH2O).

In the example shown, the valve 3500 may be configured to close when the pressure in the plenum chamber 3200 reaches a predetermined threshold which is less than therapy pressure. In one example the valve 3500 may close when the pressure reaches around 1 cmH2O. In another example, the valve 3500 may close when the pressure reaches around 2 cmH2O.

The valve 3500 may comprise a valve member 3510 (e.g. a flap made from relatively high Durometer silicone) which is biased towards an open position (e.g. inside the plenum chamber, as shown in Fig. 7A), and which is configured to close when the predetermined pressure is reached. In one form of the technology, the flap 3510 may be provided with one or more apertures 3402 which together form a vent 3400. One or more additional vents (not shown) may additionally or alternatively be provided to the plenum chamber 3200 and/or other components of the system.

In some forms of the technology the total vent area of the system may be greater than that provided to some prior art systems. In one example the total vent flow area may be twice the vent flow area required for a patient interface having the same geometry but no valve 3500 of the type shown in Figs. 7A and 7B. In one example the total vent area may be about 25 mm2. This additional vent area ensures that there is adequate $CO_2$ washout during the period in which the system pressure increases from the predetermined valve shutting pressure up to therapy pressure.

In some forms of the technology the patient interface 3000 described above with reference to Figs. 7A and 7B may be used with an RPT device 4000 which can provided a higher maximum flow rate than the RPT devices of the prior art, such that higher therapy pressures can be maintained (if required) despite the additional venting area.

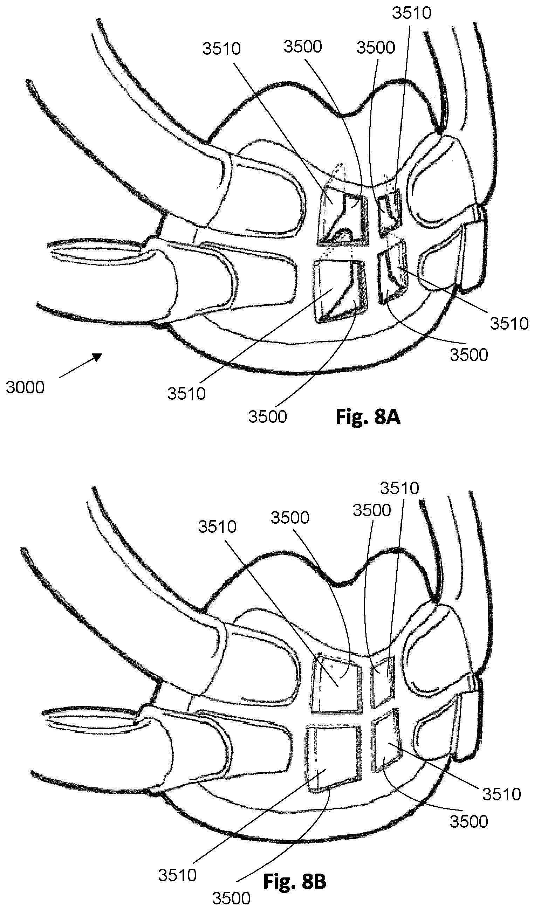

Referring next to Figs. 8a and 8b, in another form of the technology the patient interface 3000 may be provided with a plurality of valves 3500. In one example the patient interface may be provided with four valves 3500.

In one example the four valves 3500 may have a combined flow area (e.g. the sum of the flow areas of all four valves 3500, when each of the valves is fully open) of about 330 mm2, although, as is noted above, the actual area required may change depending on the geometry of the patient interface. In examples, each of the valves 3500 has substantially the same flow area, although in some examples one or more of the valves 3500 may have a different flow area to one or more of the other valves 3500.

Each valve 3500 may comprise a valve member 3510, e.g. a flap, which is biased towards an open position (e.g. with the flap inside the plenum chamber). In examples, each of the valves 3500 is configured to close at a different pressure. For example, a first of the valves 3500 may close when the pressure in the patient interface reaches 1 cmH2O. A second of the valves 3500 may close when the pressure reaches 2 cmH2O. A third of the valves 3500 may close when the pressure reaches 3 mmH2O. A fourth one of the valves 3500 may close when the pressure reaches around 4 mmH2O. The valves may be configured to close at different pressures by, for example, varying material hardness of the valve members 3510 and/or living hinge relief size.

In this way, adequate flow rates for CO2 washout can be achieved at very low plenum chamber pressures, and the pressure within the plenum chamber 3200 can be increased gradually up to normal therapy pressure. Examples such as those shown in Figs. 8A and 8B may not require an unusually large venting area, such as may be required with the example described above with reference to Figs. 7A and 7B. As a consequence, when operating at normal therapy pressures the RPT device may only need to provide flow rates which are similar to or the same as those of prior art RPT devices.

The construction of each of the valves 3500 referred to above may be similar to that of traditional anti-asphyxia valves (AAVs), with suitable modifications to alter the pressure at which the valves 3500 close.

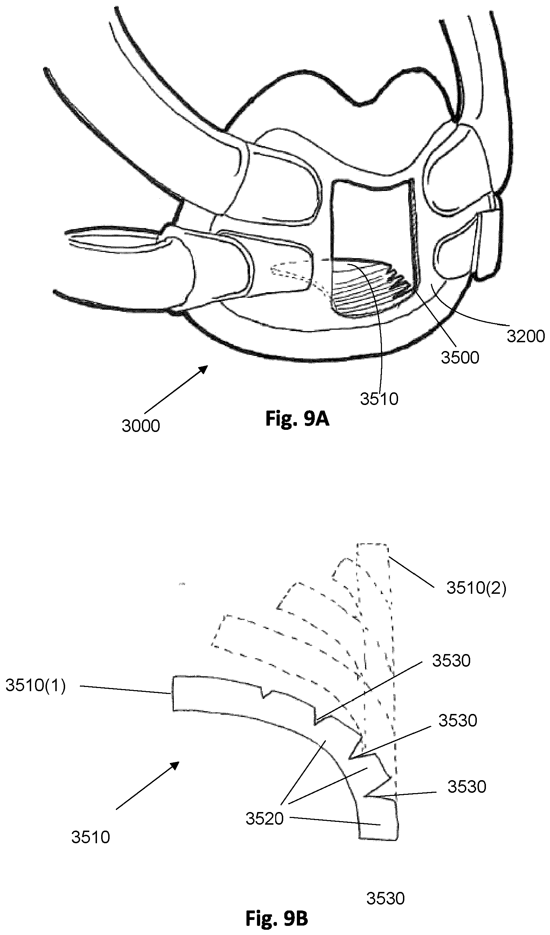

Referring next to Figs. 9A to 9C, in another example the patient interface comprises a valve 3500. The valve 3500 may be provided with a valve member 3510 (e.g. a flap) which is biased to an open position.

The flap 3510 may be configured to close progressively as the pressure within the plenum chamber 3200 increases. In one example, the flap 3500 may comprise a plurality of flap sections 3520, each of which is connected to an adjacent section by a hinge portion 3530 (e.g. a living hinge). The hinge portions 3530 may all be substantially parallel to each other.

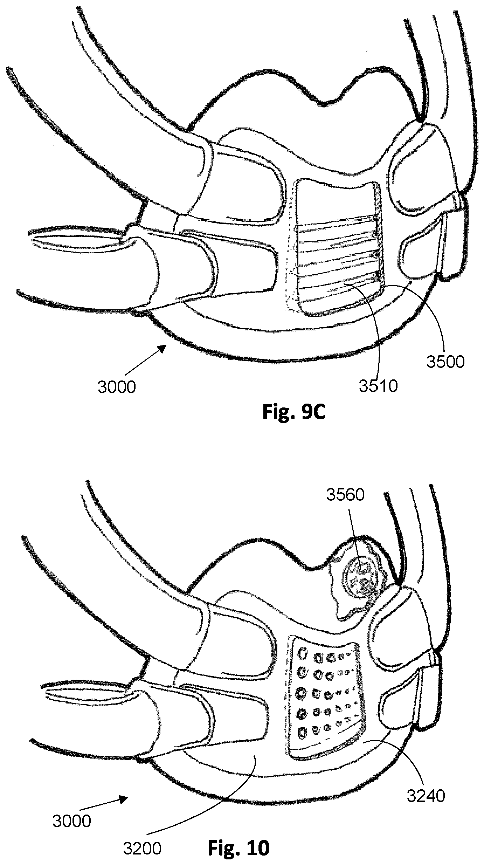

Each hinge portion 3530 may be configured to bias the adjacent flap section 3520 to an open position. The flap sections 3520 and hinge portions 3530 may be configured such that flap 3510 reduces the area available for flow through the valve 3500 in proportion to an increase in pressure in the plenum chamber 3200. Fig. 9B shows the configuration of the flap 3510 for a variety of different plenum chamber pressures, from a very low pressure configuration indicated by 3510(1) (and shown in Fig. 9A) to a closed configuration, indicated by 3510(2) (and shown in Figs. 9B and 9C), when the pressure is at, or greater than, a minimum therapy pressure.

In one example, each hinge portion 3530 is a living hinge, and the thickness of the hinge portions 3530 increases along the length of the flap (e.g with increasing distance from the base 3540 of the flap 3510), e.g. such that the hinge portion 3530 which is the greatest distance from the base 3450 is thickest (and therefore has the greatest resistance to closing), and the hinge portion 3530 closest to the base 3540 is thinnest (and therefore is configured to close at the lower pressure than the other hinge portions 3530).

As with the example described above with refence to Figs. 8A and 8B, this example may allow adequate flow rates for CO2 washout at very low plenum chamber pressures, as well as allowing the pressure within the plenum chamber 3200 to be increased gradually up to normal therapy pressure. Normal vent flow areas and RPT device flow rates can be used.

### 4.3.5.1 Soft closing valves

Typical AAV valves may emit a sound and/or create a vibration when moving from an open configuration to a closed configuration.

By contrast, each of the valves 3500 described above with reference to Figs. 7A to 9C may be configured to close relatively quietly and/or softly.

In examples, each valve 3500 may comprise a valve seat portion 3550 (see Fig. 7A) against which the valve flap 3510 seals when closed. In some forms of the technology the valve seat portion 3550 may comprise a relatively soft material such as relatively low Durometer silicone and/or foam. In other examples, a soft material may be provided to at least the periphery of the valve flap 3510, e.g. where the valve flap 3510 contacts the seat portion 3550 when closed.

In some examples (e.g. the example shown in Fig. 9A-9C) it may not be necessary to provide such a soft valve seat portion 3550, as the valve flap 3510 may close sufficiently slowly that no, or very little, sound and/or vibration is created.

In other examples, one or more electrically actuated valves (not shown) may be provided, for example such as those described in US Patent Publication US20220401681A1 (in particular those shown in Figs. 12-23), the contents of which is included herein by reference. Such valves 3500 may be controllable to close with relatively little noise or vibration. Such valves 3500 may also be controllable to vary the resistance to flow through the valve 3500 (e.g. by decreasing available flow area) in proportion to increasing pressure in the plenum chamber 3200.

### 4.3.6 In-mask sensor

Referring next to Fig. 10, in some forms of the technology a patient interface 3000 may be provided with one or more sensors 3560 which sense a characteristic of the air within the plenum chamber 3200. The one or more sensors 3560 may include one or more of: a flow sensor; a pressure sensor; a CO2 sensor and a VOC sensor. The sensor(s) 3560 may provide information to the RPT device (or another associated processor) to assist in ascertaining whether the patient has fallen asleep.

In other examples, one or more of the sensors 3560 mentioned above may be provided to the outlet end of the air circuit 4170, as described further below.

In some forms of the technology the patient interface 3000 may be provided with at least one accelerometer, to allow the system to determine the patient's sleeping position, e.g. whether the patient is sleeping on their back or on their side.

### 4.3.7 Decoupling structure(s)

In one form the patient interface 3000 includes at least one decoupling structure, for example, a swivel or a ball and socket.

### 4.3.8 Connection port

Connection port 3600 allows for connection to the air circuit 4170.

### 4.3.9 Forehead support

In one form, the patient interface 3000 includes a forehead support 3700.

### 4.3.10 Anti-asphyxia valve

In one form, the patient interface 3000 includes an anti-asphyxia valve.

### 4.3.11 Ports

In one form of the present technology, a patient interface 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

### 4.4 SLEEP DETECTION AT LOW PRESSURE

As is mentioned above, examples of the technology include methods of providing therapy in which the pressure supplied to the patient interface 3000 is maintained at a very low level (e.g. less than 1 cmH2O, less than 0.5 cmH2O or less than 0.2 cmH2O) until the patient is asleep.

Some prior art methods of detecting sleep in a patient may not suitable at such low pressures. For example, ResMed's "AutoRamp" system determines that a patient is asleep if it detects one of the following:

- 30 breaths of stable breathing (roughly 3 minutes);

- 5 consecutive snore breaths; or

- 3 obstructive apnoeas or hypopneas within 2 minutes.

These events cause pressure fluctuations which are passed down the air flow in the air circuit and are detected by sensors within the RPT device 4000. However, in examples in which the pressure is maintained at a very low level (e.g. with large open valve areas such the patient interfaces described in Figs. 7-9), the pressure "signal" which is transmitted down the air circuit 4170 may be too weak to allow reliable detection of sleep. Difficulties may become apparent at pressures of about 2cmH2O or below.

A number of solutions to this problem are possible, some of which are set out below.

### 4.4.1 Additional sensor(s)

In one form of the technology a patient interface 3000 may be provided with one or more sensors 3560 which sense a characteristic of the air within the plenum chamber 3200 which can be correlated to the patient's breathing. The one or more sensors 3560 may include one or more of: a flow sensor; a pressure sensor; a CO2 sensor and a VOC sensor. The sensor(s) 3560 may provide information to the RPT device 4000 (or another associated processor) to assist in ascertaining whether the patient has fallen asleep.

In other examples, one or more of the sensors 3560 mentioned above may be provided to the outlet end of the air circuit 4170, that is, the end of the air circuit 4170 which is connected to the patient interface 3000 in use.

### 4.4.2 Alternative sleep detection methods

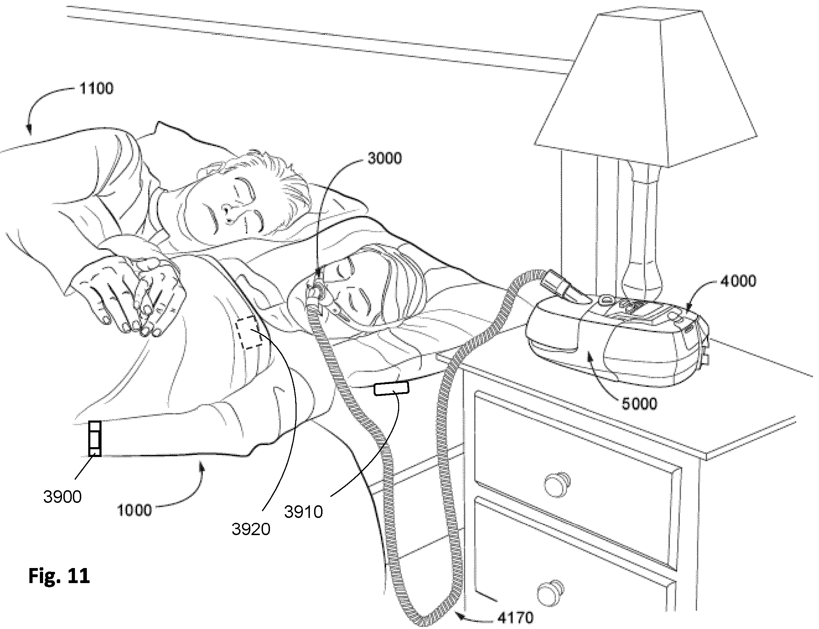

Referring next to Fig. 11, in one form of the technology, the patient 1000 may wear a smart watch or other similar wearable device 3900 (e.g. a fitness tracker or a bespoke wearable sensor) which can detect movement (e.g. via one or more accelerometers). The wearable device 3900 may be in communication with the RPT device 4000 (or other associated processor), for example via WI-FI or Bluetooth. The wearable 3900 may signal to the RPT device 4000 or processor when the patient 1000 has fallen asleep (based on on-board analysis of the patient's movement) and/or may simply indicate whenever the patient 1000 moves, such that the RPT device 4000 or processor can determine when the patient 1000 is asleep.

In another example, the information may be provided by a smart phone or similar 3910, e.g. one which is positioned beneath a user's pillow.

In another example, a patient interface may comprise a suitable number of of EEG sensors (for example, integrated into the patient interface headgear) which are configured to allow detection of sleep by monitoring the patient's brain waves.

## 4.5 CESSATION OF THERAPY PRESSURE

In addition to having difficulty in going to sleep while receiving pressurised air, some patients may also dislike the feeling of waking up while receiving pressurised air. In some examples of the prior art, the patient may wake to find their patient interface is pressurised at full therapy pressure, rather than the reduced (but still elevated) pressure supplied by many systems before sleep is detected.

In one form of the present technology, the system may move to operating in the "Breathe Free" mode when the system detects that the patient has woken up, or, in some cases, prior to the patient waking up.

In examples, any of the methods described above for detecting when the patient is asleep may also be used to detect that the patient is awake.

In some forms of the technology the patient may program the RPT device 4000 with a predetermined time (e.g. 15 minutes prior to a morning alarm sounding) at which therapy pressure should be ceased. In some forms of technology, the RPT device 4000 (and/or additional processor) may use machine learning or similar to predict when the patient is in a light sleep phase and/or is likely to wake within a predetermined time in the future (e.g. about 12 minutes or less), to thereby ensure that the patient is only receiving the very low "Breathe Free" pressure at that time. In some examples, the machine learning may receive information from a wearable 3900 or similar device which is worn by the patient when asleep.

In some forms of technology, e.g. for use when the patient 1000 suffers from OSA and/or snoring predominately or only when lying in a certain position/orientation (e.g. on their back) the RPT device 4000 may use signals from a wearable 3900 or similar device (e.g. a device 3920 comprising at least one accelerometer which is engaged with the patient's chest), and/or from a sensor provided to the patient interface 3000, to detect when the patient 1000 is lying on their side, and may move to the Breath Free mode whenever this is the case. The RPT device 4000 may increase the pressure to therapy pressure only when the patient 1000 is lying on their back.

## 4.6 SEAL TEST PROCEDURE

In examples of the invention, the patient is only ever subject to very low pressure while trying to fall asleep. However, such low pressure may not be sufficient to reveal whether the patient interface 3000 will leak when exposed to the much higher therapy pressure.

In order to ensure that the patient interface 3000 will not leak when exposed to the therapy pressure, the RPT device 4000 may be configured to operate in a test cycle mode. In such a mode the RPT device 4000 may increase the pressure up to therapy pressure over a relatively short period, e.g. less than two minutes, e.g. about one minute, beginning at a pressure which is less than therapy pressure, e.g. less than 4 cmH2O, or less than 1 cmH2O. If any leak is detected the pressure increase may be paused and the patient 1000 may be prompted to make an adjustment of the patient interface 3000 and/or headgear 3300. The pressure increase may then be resumed.

Once therapy pressure is reached with no leak detected, the test cycle may end and the system may reset to the Breathe Free setting until the patient is asleep, after which the pressure may be increased to a therapy pressure, as described further herein.

## 4.7 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 cmH2O, or at least 10cmH2O, or at least 20 cmH2O.

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more air path items, e.g., an inlet air filter 4112, an inlet muffler 4122, a pressure generator 4140 capable of supplying air at positive pressure (e.g., a blower 4142), an outlet muffler 4124 and one or more transducers 4270, such as pressure sensors 4272 and flow rate sensors 4274.

One or more of the air path items may be located within a removable unitary structure which will be referred to as a pneumatic block 4020. The pneumatic block 4020 may be located within the external housing 4010. In one form a pneumatic block 4020 is supported by, or formed as part of the chassis 4016.

The RPT device 4000 may have an electrical power supply 4210, one or more input devices 4220, a central controller 4230, a therapy device controller 4240, a pressure generator 4140, one or more protection circuits 4250, memory 4260, transducers 4270, data communication interface 4280 and one or more output devices 4290. Electrical components 4200 may be mounted on a single Printed Circuit Board Assembly (PCBA) 4202. In an alternative form, the RPT device 4000 may include more than one PCBA 4202.

### 4.7.1 RPT device mechanical & pneumatic components

An RPT device may comprise one or more of the following components in an integral unit. In an alternative form, one or more of the following components may be located as respective separate units.

### 4.7.1.1 Air filter(s)

An RPT device in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3000 or 3800.

### 4.7.1.2 Muffler(s)

An RPT device in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3000 or 3800.

### 4.7.1.3 Pressure generator

In one form of the present technology, a pressure generator 4140 for producing a flow, or a supply, of air at positive pressure is a controllable blower 4142. For example, the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 cmH2O to about 20 cmH2O, or in other forms up to about 30 cmH2O when delivering respiratory pressure therapy. The blower may be as described in any one of the following patents or patent applications U.S. Patent No. 7,866,944; U.S. Patent No. 8,638,014; U.S. Patent No. 8,636,479; and PCT Patent Application Publication No. WO 2013/020167.

The pressure generator 4140 may be under the control of the therapy device controller 4240.

In other forms, a pressure generator 4140 may be a piston-driven pump, a pressure regulator connected to a high pressure source (e.g. compressed air reservoir), or a bellows.

### 4.7.1.4 Transducer(s)

Transducers may be internal of the RPT device, or external of the RPT device. External transducers may be located for example on or form part of the air circuit, e.g., the patient interface. External transducers may be in the form of noncontact sensors such as a Doppler radar movement sensor that transmit or transfer data to the RPT device.

In one form of the present technology, one or more transducers 4270 are located upstream and/or downstream of the pressure generator 4140. The one or more transducers 4270 may be constructed and arranged to generate signals representing properties of the flow of air such as a flow rate, a pressure or a temperature at that point in the pneumatic path.

In one form of the present technology, one or more transducers 4270 may be located proximate to the patient interface 3000 or 3800.

In one form, a signal from a transducer 4270 may be filtered, such as by low-pass, high-pass or band-pass filtering.

#### 4.7.1.4.1 Flow rate sensor

A flow rate sensor 4274 in accordance with the present technology may be based on a differential pressure transducer, for example, an SDP600 Series differential pressure transducer from SENSIRION.

In one form, a signal generated by the flow rate sensor 4274 and representing a flow rate is received by the central controller 4230.

#### 4.7.1.4.2 Pressure sensor

A pressure sensor 4272 in accordance with the present technology is located in fluid communication with the pneumatic path. An example of a suitable pressure sensor is a transducer from the HONEYWELL ASDX series. An alternative suitable pressure sensor is a transducer from the NPA Series from GENERAL ELECTRIC.

In one form, a signal generated by the pressure sensor 4272 and representing a pressure is received by the central controller 4230.

#### 4.7.1.4.3 Motor speed transducer

In one form of the present technology a motor speed transducer 4276 is used to determine a rotational velocity of the motor 4144 and/or the blower 4142. A motor speed signal from the motor speed transducer 4276 may be provided to the therapy device controller 4240. The motor speed transducer 4276 may, for example, be a speed sensor, such as a Hall effect sensor.

### 4.7.1.5 Anti-spill back valve

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 4.7.2 RPT device electrical components

### 4.7.2.1 Power supply

A power supply 4210 may be located internal or external of the external housing 4010 of the RPT device 4000.

In one form of the present technology, power supply 4210 provides electrical power to the RPT device 4000 only. In another form of the present technology, power supply 4210 provides electrical power to both RPT device 4000 and humidifier 5000.

### 4.7.2.2 Input devices

In one form of the present technology, an RPT device 4000 includes one or more input devices 4220 in the form of buttons, switches or dials to allow a person to interact with the device. The buttons, switches or dials may be physical devices, or software devices accessible via a touch screen. The buttons, switches or dials may, in one form, be physically connected to the external housing 4010, or may, in another form, be in wireless communication with a receiver that is in electrical connection to the central controller 4230.

In one form, the input device 4220 may be constructed and arranged to allow a person to select a value and/or a menu option.

### 4.7.2.3 Central controller

In one form of the present technology, the central controller 4230 is one or a plurality of processors suitable to control an RPT device 4000.

Suitable processors may include an x86 INTEL processor, a processor based on ARM® Cortex®-M processor from ARM Holdings such as an STM32 series microcontroller from ST MICROELECTRONIC. In certain alternative forms of the present technology, a 32-bit RISC CPU, such as an STR9 series microcontroller from ST MICROELECTRONICS or a 16-bit RISC CPU such as a processor from the MSP430 family of microcontrollers, manufactured by TEXAS INSTRUMENTS may also be suitable.

In one form of the present technology, the central controller 4230 is a dedicated electronic circuit.

In one form, the central controller 4230 is an application-specific integrated circuit. In another form, the central controller 4230 comprises discrete electronic components.

The central controller 4230 may be configured to receive input signal(s) from one or more transducers 4270, one or more input devices 4220, and the humidifier 5000.

The central controller 4230 may be configured to provide output signal(s) to one or more of an output device 4290, a therapy device controller 4240, a data communication interface 4280, and the humidifier 5000.

In some forms of the present technology, the central controller 4230 is configured to implement the one or more methodologies described herein, such as the one or more algorithms 4300 which may be implemented with processor-control instructions, expressed as computer programs stored in a non-transitory computer readable storage medium, such as memory 4260. In some forms of the present technology, the central controller 4230 may be integrated with an RPT device 4000. However, in some forms of the present technology, some methodologies may be performed by a remotely located device. For example, the remotely located device may determine control settings for a ventilator or detect respiratory related events by analysis of stored data such as from any of the sensors described herein.

### 4.7.2.4 Clock

The RPT device 4000 may include a clock 4232 that is connected to the central controller 4230.

### 4.7.2.5 Therapy device controller

In one form of the present technology, therapy device controller 4240 is a therapy control module 4330 that forms part of the algorithms 4300 executed by the central controller 4230.

In one form of the present technology, therapy device controller 4240 is a dedicated motor control integrated circuit. For example, in one form a MC33035 brushless DC motor controller, manufactured by ONSEMI is used.

### 4.7.2.6 Protection circuits

The one or more protection circuits 4250 in accordance with the present technology may comprise an electrical protection circuit, a temperature and/or pressure safety circuit.

### 4.7.2.7 Memory

In accordance with one form of the present technology the RPT device 4000 includes memory 4260, e.g., non-volatile memory. In some forms, memory 4260 may include battery powered static RAM. In some forms, memory 4260 may include volatile RAM.

Memory 4260 may be located on the PCBA 4202. Memory 4260 may be in the form of EEPROM, or NAND flash.

Additionally, or alternatively, RPT device 4000 includes a removable form of memory 4260, for example a memory card made in accordance with the Secure Digital (SD) standard.

In one form of the present technology, the memory 4260 acts as a non-transitory computer readable storage medium on which is stored computer program instructions expressing the one or more methodologies described herein, such as the one or more algorithms 4300.

### 4.7.2.8 Data communication systems

In one form of the present technology, a data communication interface 4280 is provided, and is connected to the central controller 4230. Data communication interface 4280 may be connectable to a remote external communication network 4282 and/or a local external communication network 4284. The remote external communication network 4282 may be connectable to a remote external device 4286. The local external communication network 4284 may be connectable to a local external device 4288.

In one form, data communication interface 4280 is part of the central controller 4230. In another form, data communication interface 4280 is separate from the central controller 4230, and may comprise an integrated circuit or a processor.

In one form, remote external communication network 4282 is the Internet. The data communication interface 4280 may use wired communication (e.g. via Ethernet, or optical fibre) or a wireless protocol (e.g. CDMA, GSM, LTE) to connect to the Internet.

In one form, local external communication network 4284 utilises one or more communication standards, such as Bluetooth, or a consumer infrared protocol.

In one form, remote external device 4286 is one or more computers, for example a cluster of networked computers. In one form, remote external device 4286 may be virtual computers, rather than physical computers. In either case, such a remote external device 4286 may be accessible to an appropriately authorised person such as a clinician.

The local external device 4288 may be a personal computer, mobile phone, tablet or remote control.

### 4.7.2.9 Output devices including optional display, alarms

An output device 4290 in accordance with the present technology may take the form of one or more of a visual, audio and haptic unit. A visual display may be a Liquid Crystal Display (LCD) or Light Emitting Diode (LED) display.

#### *4.7.2.9.1 Display driver*

A display driver 4292 receives as an input the characters, symbols, or images intended for display on the display 4294, and converts them to commands that cause the display 4294 to display those characters, symbols, or images.

#### *4.7.2.9.2 Display*

A display 4294 is configured to visually display characters, symbols, or images in response to commands received from the display driver 4292. For example, the display 4294 may be an eight-segment display, in which case the display driver 4292 converts each character or symbol, such as the figure "0", to eight logical signals indicating whether the eight respective segments are to be activated to display a particular character or symbol.

### 4.7.3 RPT device algorithms

As mentioned above, in some forms of the present technology, the central controller 4230 may be configured to implement one or more algorithms 4300 expressed as computer programs stored in a non-transitory computer readable storage medium, such as memory 4260. The algorithms 4300 are generally grouped into groups referred to as modules.

In other forms of the present technology, some portion or all of the algorithms 4300 may be implemented by a controller of an external device such as the local external device 4288 or the remote external device 4286. In such forms, data representing the input signals and / or intermediate algorithm outputs necessary for the portion of the algorithms 4300 to be executed at the external device may be communicated to the external device via the local external communication network 4284 or the remote external communication network 4282. In such forms, the portion of the algorithms 4300 to be executed at the external device may be expressed as computer programs, such as with processor control instructions to be executed by one or more processor(s), stored in a non-transitory computer readable storage medium accessible to the controller of the external device. Such programs configure the controller of the external device to execute the portion of the algorithms 4300.

In such forms, the therapy parameters generated by the external device via the therapy engine module 4320 (if such forms part of the portion of the algorithms 4300 executed by the external device) may be communicated to the central controller 4230 to be passed to the therapy control module 4330.

### 4.7.3.1 Pre-processing module

A pre-processing module 4310 in accordance with one form of the present technology receives as an input a signal from a transducer 4270, for example a flow rate sensor 4274 or pressure sensor 4272, and performs one or more process steps to calculate one or more output values that will be used as an input to another module, for example a therapy engine module 4320.

In one form of the present technology, the output values include the interface pressure $Pm$, the vent flow rate $Qv$,

the respiratory flow rate $Qr$, and the leak flow rate $Ql$.

In various forms of the present technology, the pre-processing module 4310 comprises one or more of the following algorithms: interface pressure estimation 4312, vent flow rate estimation 4314, leak flow rate estimation 4316, and respiratory flow rate estimation 4318.

### 4.7.3.1.1 Interface pressure estimation

In one form of the present technology, an interface pressure estimation algorithm 4312 receives as inputs a signal from the pressure sensor 4272 indicative of the pressure in the pneumatic path proximal to an outlet of the pneumatic block (the device pressure Pd) and a signal from the flow rate sensor 4274 representative of the flow rate of the airflow leaving the RPT device 4000 (the device flow rate $Qd$). The device flow rate $Qd$, absent any supplementary gas 4180, may be used as the total flow rate $Qt$. The interface pressure algorithm 4312 estimates the pressure drop $\Delta P$ through the air circuit 4170. The dependence of the pressure drop $\Delta P$ on the total flow rate $Qt$ may be modelled for the particular air circuit 4170 by a pressure drop characteristic $\Delta P(Q)$. The interface pressure estimation algorithm, 4312 then provides as an output an estimated pressure, $Pm$, in the patient interface 3000 or 3800. The pressure, $Pm$, in the patient interface 3000 or 3800 may be estimated as the device pressure $Pd$ minus the air circuit pressure drop $\Delta P$.

### 4.7.3.1.2 Vent flow rate estimation

In one form of the present technology, a vent flow rate estimation algorithm 4314 receives as an input an estimated pressure, $Pm$, in the patient interface 3000 or 3800 from the interface pressure estimation algorithm 4312 and estimates a vent flow rate of air, $Qv$, from a vent 3400 in a patient interface 3000 or 3800. The dependence of the vent flow rate $Qv$ on the interface pressure $Pm$ for the particular vent 3400 in use may be modelled by a vent characteristic $Qv(Pm)$.

### 4. 7.3.1.3 Leak flow rate estimation

In one form of the present technology, a leak flow rate estimation algorithm 4316 receives as an input a total flow rate, $Qt$, and a vent flow rate $Qv$, and provides as an output an estimate of the leak flow rate $Ql$. In one form, the leak flow rate estimation algorithm estimates the leak flow rate $Ql$ by calculating an average of the difference between total flow rate $Qt$ and vent flow rate $Qv$ over a period sufficiently long to include several breathing cycles, e.g. about 10 seconds.

In one form, the leak flow rate estimation algorithm 4316 receives as an input a total flow rate $Qt$, a vent flow rate $Qv$, and an estimated pressure, $Pm$, in the patient interface 3000 or 3800, and provides as an output a leak flow rate $Ql$, by calculating a leak conductance, and determining a leak flow rate $Ql$ to be a function of leak conductance and pressure, $Pm$. Leak conductance is calculated as the quotient of low pass filtered non-vent flow rate equal to the difference between total flow rate $Qt$ and vent flow rate $Qv$, and low pass filtered square root of pressure $Pm$, where the low pass filter time constant has a value sufficiently long to include several breathing cycles, e.g. about 10 seconds. The leak flow rate $Ql$ may be estimated as the product of leak conductance and a function of pressure, $Pm$.

### 4.7.3.1.4 Respiratory flow rate estimation

In one form of the present technology, a respiratory flow rate estimation algorithm 4318 receives as an input a total flow rate, $Qt$, a vent flow rate, $Qv$, and a leak flow rate, $Ql$, and estimates a respiratory flow rate of air, $Qr$, to the patient, by subtracting the vent flow rate $Qv$ and the leak flow rate $Ql$ from the total flow rate $Qt$.

### 4.7.3.2 Therapy Engine Module

In one form of the present technology, a therapy engine module 4320 receives as inputs one or more of a pressure, $Pm$, in a patient interface 3000 or 3800, and a respiratory flow rate of air to a patient, $Qr$, and provides as an output one or more therapy parameters.

In one form of the present technology, a therapy parameter is a treatment pressure $Pt$.

In one form of the present technology, therapy parameters are one or more of an amplitude of a pressure variation, a base pressure, and a target ventilation.

In various forms, the therapy engine module 4320 comprises one or more of the following algorithms: phase determination 4321, waveform determination 4322, ventilation determination 4323, inspiratory flow limitation determination 4324, apnea / hypopnea determination 4325, snore determination 4326, airway patency determination 4327, target ventilation determination 4328, and therapy parameter determination 4329.

### 4.7.3.2.1 Phase determination

In one form of the present technology, the RPT device 4000 does not determine phase.

In one form of the present technology, a phase determination algorithm 4321 receives as an input a signal indicative of respiratory flow rate, $Qr$, and provides as an output a phase $\Phi$ of a current breathing cycle of a patient 1000.

In some forms, known as discrete phase determination, the phase output $\Phi$ is a discrete variable. One implementation of discrete phase determination provides a bi-valued phase output $\Phi$ with values of either inhalation or exhalation, for example represented as values of 0 and 0.5 revolutions respectively, upon detecting the start of spontaneous inhalation and exhalation respectively. RPT devices 4000 that "trigger" and "cycle" effectively perform discrete phase determination, since the trigger and cycle points are the instants at which the phase changes from exhalation to inhalation and from inhalation to exhalation, respectively. In one implementation of bi-valued phase determination, the phase output $\Phi$ is determined to have a discrete value of 0 (thereby "triggering" the RPT device 4000) when the respiratory flow rate $Qr$ has a value that exceeds a positive threshold, and a discrete value of 0.5 revolutions (thereby "cycling" the RPT device 4000) when a respiratory flow rate $Qr$ has a value that is more negative than a negative threshold. The inhalation time $Ti$ and the exhalation time $Te$ may be estimated as typical values over many respiratory cycles of the time spent with phase $\Phi$ equal to 0 (indicating inspiration) and 0.5 (indicating expiration) respectively.

Another implementation of discrete phase determination provides a tri-valued phase output $\Phi$ with a value of one of inhalation, mid-inspiratory pause, and exhalation.

In other forms, known as continuous phase determination, the phase output $\Phi$ is a continuous variable, for example varying from 0 to 1 revolutions, or 0 to $2\pi$ radians. RPT devices 4000 that perform continuous phase determination may trigger and cycle when the continuous phase reaches 0 and 0.5 revolutions, respectively. In one implementation of continuous phase determination, a continuous value of phase $\Phi$ is determined using a fuzzy logic analysis of the respiratory flow rate $Qr$. A continuous value of phase determined in this implementation is often referred to as "fuzzy phase". In one implementation of a fuzzy phase determination algorithm 4321, the following rules are applied to the respiratory flow rate $Qr$:

1. If $Qr$ is zero and increasing fast then $\Phi$ is 0 revolutions.

2. If $Qr$ is large positive and steady then $\Phi$ is 0.25 revolutions.

3. If $Qr$ is zero and falling fast, then $\Phi$ is 0.5 revolutions.

4. If $Qr$ is large negative and steady then $\Phi$ is 0.75 revolutions.

5. If $Qr$ is zero and steady and the 5-second low-pass filtered absolute value of $Qr$ is large then $\Phi$ is 0.9 revolutions.

6. If $Qr$ is positive and the phase is expiratory, then $\Phi$ is 0 revolutions.

7. If $Qr$ is negative and the phase is inspiratory, then $\Phi$ is 0.5 revolutions.

8. If the 5-second low-pass filtered absolute value of $Qr$ is large, $\Phi$ is increasing at a steady rate equal to the patient's breathing rate, low-pass filtered with a time constant of 20 seconds.

The output of each rule may be represented as a vector whose phase is the result of the rule and whose magnitude is the fuzzy extent to which the rule is true. The fuzzy extent to which the respiratory flow rate is "large", "steady", etc. is determined with suitable membership functions. The results of the rules, represented as vectors, are then combined by some function such as taking the centroid. In such a combination, the rules may be equally weighted, or differently weighted.

In another implementation of continuous phase determination, the phase $\Phi$ is first discretely estimated from the respiratory flow rate $Qr$ as described above, as are the inhalation time $Ti$ and the exhalation time $Te$. The continuous phase $\Phi$ at any instant may be determined as the half the proportion of the inhalation time $Ti$ that has elapsed since the previous trigger instant, or 0.5 revolutions plus half the proportion of the exhalation time $Te$ that has elapsed since the previous cycle instant (whichever instant was more recent).

### 4.7.3.2.2 Waveform determination

In one form of the present technology, the therapy parameter determination algorithm 4329 provides an approximately constant treatment pressure throughout a respiratory cycle of a patient.

In other forms of the present technology, the therapy control module 4330 controls the pressure generator 4140 to provide a treatment pressure *Pt* that varies as a function of phase Φ of a respiratory cycle of a patient according to a waveform template Π(Φ).

In one form of the present technology, a waveform determination algorithm 4322 provides a waveform template Π(Φ) with values in the range [0, 1] on the domain of phase values Φ provided by the phase determination algorithm 4321 to be used by the therapy parameter determination algorithm 4329.

In one form, suitable for either discrete or continuously-valued phase, the waveform template Π(Φ) is a square-wave template, having a value of 1 for values of phase up to and including 0.5 revolutions, and a value of 0 for values of phase above 0.5 revolutions. In one form, suitable for continuously-valued phase, the waveform template Π(Φ) comprises two smoothly curved portions, namely a smoothly curved (e.g. raised cosine) rise from 0 to 1 for values of phase up to 0.5 revolutions, and a smoothly curved (e.g. exponential) decay from 1 to 0 for values of phase above 0.5 revolutions. In one form, suitable for continuously-valued phase, the waveform template Π(Φ) is based on a square wave, but with a smooth rise from 0 to 1 for values of phase up to a "rise time" that is less than 0.5 revolutions, and a smooth fall from 1 to 0 for values of phase within a "fall time" after 0.5 revolutions, with a "fall time" that is less than 0.5 revolutions.

In some forms of the present technology, the waveform determination algorithm 4322 selects a waveform template Π(Φ) from a library of waveform templates, dependent on a setting of the RPT device. Each waveform template Π(Φ) in the library may be provided as a lookup table of values Π against phase values Φ. In other forms, the waveform determination algorithm 4322 computes a waveform template Π(Φ) "on the fly" using a predetermined functional form, possibly parametrised by one or more parameters (e.g. time constant of an exponentially curved portion). The parameters of the functional form may be predetermined or dependent on a current state of the patient 1000.

In some forms of the present technology, suitable for discrete bi-valued phase of either inhalation (Φ = 0 revolutions) or exhalation (Φ = 0.5 revolutions), the waveform determination algorithm 4322 computes a waveform template Π "on the fly" as a function of both discrete phase Φ and time t measured since the most recent trigger instant. In one such form, the waveform determination algorithm 4322 computes the waveform template Π(Φ, t) in two portions (inspiratory and expiratory) as follows:

$$\Pi(\Phi, t) = \begin{cases} \Pi_i(t), & \Phi = 0 \\ \Pi_e(t - T_i), & \Phi = 0.5 \end{cases}$$

where $\Pi_i(t)$ and $\Pi_e(t)$ are inspiratory and expiratory portions of the waveform template Π(Φ, t). In one such form, the inspiratory portion $\Pi_i(t)$ of the waveform template is a smooth rise from 0 to 1 parametrised by a rise time, and the expiratory portion $\Pi_e(t)$ of the waveform template is a smooth fall from 1 to 0 parametrised by a fall time.

### 4.7.3.2.3 Ventilation determination

In one form of the present technology, a ventilation determination algorithm 4323 receives an input a respiratory flow rate *Qr,* and determines a measure indicative of current patient ventilation, *Vent.*

In some implementations, the ventilation determination algorithm 4323 determines a measure of ventilation *Vent* that is an estimate of actual patient ventilation. One such implementation is to take half the absolute value of respiratory flow rate, *Qr,* optionally filtered by low-pass filter such as a second order Bessel low-pass filter with a corner frequency of 0.11 Hz.

In other implementations, the ventilation determination algorithm 4323 determines a measure of ventilation *Vent* that is broadly proportional to actual patient ventilation. One such implementation estimates peak respiratory flow rate *Qpeak* over the inspiratory portion of the cycle. This and many other procedures involving sampling the respiratory flow rate *Qr* produce measures which are broadly proportional to ventilation, provided the flow rate waveform shape does not vary very much (here, the shape of two breaths is taken to be similar when the flow rate waveforms of the breaths normalised in time and amplitude are similar). Some simple examples include the median positive respiratory flow rate, the median of the absolute value of respiratory flow rate, and the standard deviation of flow rate. Arbitrary linear combinations of arbitrary order statistics of the absolute value of respiratory flow rate using positive coefficients, and even some using both positive and negative coefficients, are approximately proportional to ventilation. Another example is the mean of the respiratory flow rate in the middle K proportion (by time) of the inspiratory portion, where $0 < K < 1$. There is an arbitrarily large number of measures that are exactly proportional to ventilation if the flow rate shape is constant.

### 4. 7.3.2.4 Determination of Inspiratory Flow Limitation

In one form of the present technology, the central controller 4230 executes an inspiratory flow limitation determination algorithm 4324 for the determination of the extent of inspiratory flow limitation.

In one form, the inspiratory flow limitation determination algorithm 4324 receives as an input a respiratory flow rate signal *Qr* and provides as an output a metric of the extent to which the inspiratory portion of the breath exhibits inspiratory flow limitation.

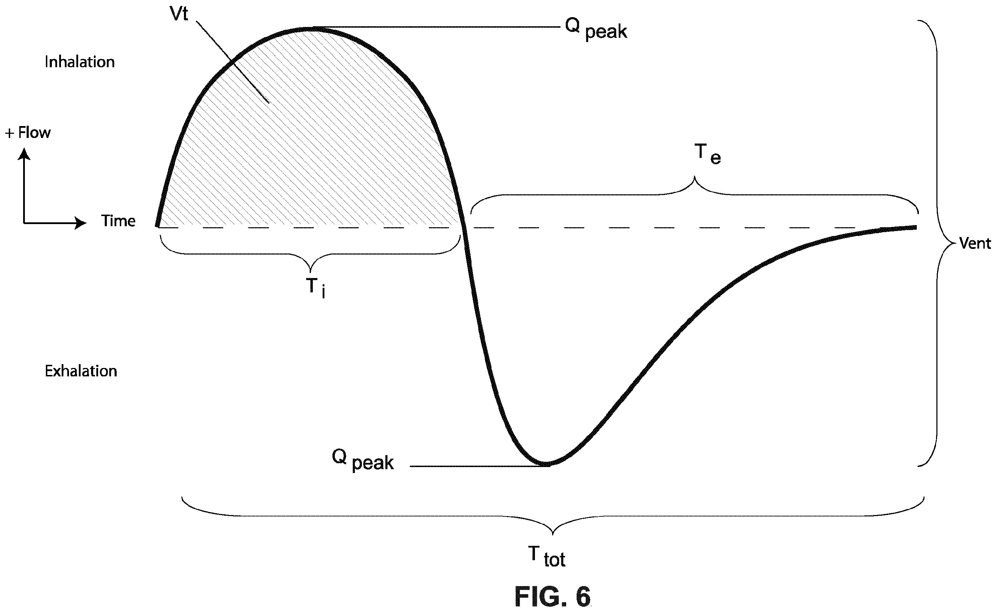

In one form of the present technology, the inspiratory portion of each breath is identified by a zero-crossing detector. A number of evenly spaced points (for example, sixty-five), representing points in time, are interpolated by an interpolator along the inspiratory flow rate-time curve for each breath. The curve described by the points is then scaled by a scalar to have unity length (duration/period) and unity area to remove the effects of changing breathing rate and depth. The scaled breaths are then compared in a comparator with a pre-stored template representing a normal unobstructed breath, similar to the inspiratory portion of the breath shown in Fig. 6. Breaths deviating by more than a specified threshold (typically 1 scaled unit) at any time during the inspiration from this template, such as those due to coughs, sighs, swallows and hiccups, as determined by a test element, are rejected. For non-rejected data, a moving average of the first such scaled point is calculated by the central controller 4230 for the preceding several inspiratory events. This is repeated over the same inspiratory events for the second such point, and so on. Thus, for example, sixty-five scaled data points are generated by the central controller 4230, and represent a moving average of the preceding several inspiratory events, e.g., three events. The moving average of continuously updated values of the (e.g., sixty-five) points are hereinafter called the "scaled flow rate ", designated as Qs(t). Alternatively, a single inspiratory event can be utilised rather than a moving average.

From the scaled flow rate, two shape factors relating to the determination of partial obstruction may be calculated.

Shape factor 1 is the ratio of the mean of the middle (e.g. thirty-two) scaled flow rate points to the mean overall (e.g. sixty-five) scaled flow rate points. Where this ratio is in excess of unity, the breath will be taken to be normal. Where the ratio is unity or less, the breath will be taken to be obstructed. A ratio of about 1.17 is taken as a threshold between partially obstructed and unobstructed breathing, and equates to a degree of obstruction that would permit maintenance of adequate oxygenation in a typical patient.

Shape factor 2 is calculated as the RMS deviation from unit scaled flow rate, taken over the middle (e.g. thirty-two) points. An RMS deviation of about 0.2 units is taken to be normal. An RMS deviation of zero is taken to be a totally flow-limited breath. The closer the RMS deviation to zero, the breath will be taken to be more flow limited.

Shape factors 1 and 2 may be used as alternatives, or in combination. In other forms of the present technology, the number of sampled points, breaths and middle points may differ from those described above. Furthermore, the threshold values can be other than those described.

### 4.7.3.2.5 Determination of apneas and hypopneas

In one form of the present technology, the central controller 4230 executes an apnea / hypopnea determination algorithm 4325 for the determination of the presence of apneas and/or hypopneas.

In one form, the apnea / hypopnea determination algorithm 4325 receives as an input a respiratory flow rate signal *Qr* and provides as an output a flag that indicates that an apnea or a hypopnea has been detected.

In one form, an apnea will be said to have been detected when a function of respiratory flow rate *Qr* falls below a flow rate threshold for a predetermined period of time. The function may determine a peak flow rate, a relatively short-term mean flow rate, or a flow rate intermediate of relatively short-term mean and peak flow rate, for example an RMS flow rate. The flow rate threshold may be a relatively long-term measure of flow rate.

In one form, a hypopnea will be said to have been detected when a function of respiratory flow rate *Qr* falls below a second flow rate threshold for a predetermined period of time. The function may determine a peak flow, a relatively short-term mean flow rate, or a flow rate intermediate of relatively short-term mean and peak flow rate, for example an RMS flow rate. The second flow rate threshold may be a relatively long-term measure of flow rate. The second flow rate threshold is greater than the flow rate threshold used to detect apneas.

### 4.7.3.2.6 Determination of snore

In one form of the present technology, the central controller 4230 executes one or more snore determination algorithms 4326 for the determination of the extent of snore.

In one form, the snore determination algorithm 4326 receives as an input a respiratory flow rate signal *Qr* and provides as an output a metric of the extent to which snoring is present.

The snore determination algorithm 4326 may comprise the step of determining the intensity of the flow rate signal in the range of 30-300 Hz. Further, the snore determination algorithm 4326 may comprise a step of filtering the respiratory flow rate signal *Qr* to reduce background noise, e.g., the sound of airflow in the system from the blower.

### 4.7.3.2.7 Determination of airway patency

In one form of the present technology, the central controller 4230 executes one or more airway patency determination algorithms 4327 for the determination of the extent of airway patency.

In one form, the airway patency determination algorithm 4327 receives as an input a respiratory flow rate signal *Qr,* and determines the power of the signal in the frequency range of about 0.75 Hz and about 3 Hz. The presence of a peak in this frequency range is taken to indicate an open airway. The absence of a peak is taken to be an indication of a closed airway.

In one form, the frequency range within which the peak is sought is the frequency of a small forced oscillation in the treatment pressure *Pt.* In one implementation, the forced oscillation is of frequency 2 Hz with amplitude about 1 cmH$_2$O.

In one form, airway patency determination algorithm 4327 receives as an input a respiratory flow rate signal *Qr,* and determines the presence or absence of a cardiogenic signal. The absence of a cardiogenic signal is taken to be an indication of a closed airway.

### 4.7.3.2.8 Determination of target ventilation

In one form of the present technology, the central controller 4230 takes as input the measure of current ventilation, *Vent,* and executes one or more target ventilation determination algorithms 4328 for the determination of a target value *Vtgt* for the measure of ventilation.

In some forms of the present technology, there is no target ventilation determination algorithm 4328, and the target value *Vtgt* is predetermined, for example by hard-coding during configuration of the RPT device 4000 or by manual entry through the input device 4220.

In other forms of the present technology, such as adaptive servo-ventilation (ASV), the target ventilation determination algorithm 4328 computes a target value *Vtgt* from a value *Vtyp* indicative of the typical recent ventilation of the patient.

In some forms of adaptive servo-ventilation, the target ventilation *Vtgt* is computed as a high proportion of, but less than, the typical recent ventilation *Vtyp.* The high proportion in such forms may be in the range (80%, 100%), or (85%, 95%), or (87%, 92%).

In other forms of adaptive servo-ventilation, the target ventilation *Vtgt* is computed as a slightly greater than unity multiple of the typical recent ventilation *Vtyp.*

The typical recent ventilation *Vtyp* is the value around which the distribution of the measure of current ventilation *Vent* over multiple time instants over some predetermined timescale tends to cluster, that is, a measure of the central tendency of the measure of current ventilation over recent history. In one implementation of the target ventilation determination algorithm 4328, the recent history is of the order of several minutes, but in any case should be longer than the timescale of Cheyne-Stokes waxing and waning cycles. The target ventilation determination algorithm 4328 may use any of the variety of well-known measures of central tendency to determine the typical recent ventilation *Vtyp* from the measure of current ventilation, *Vent.* One such measure is the output of a low-pass filter on the measure of current ventilation *Vent,* with time constant equal to one hundred seconds.

### 4.7.3.2.9 Determination of therapy parameters

In some forms of the present technology, the central controller 4230 executes one or more therapy parameter determination algorithms 4329 for the determination of one or more therapy parameters using the values returned by one or more of the other algorithms in the therapy engine module 4320.

In one form of the present technology, the therapy parameter is an instantaneous treatment pressure *Pt.* In one implementation of this form, the therapy parameter determination algorithm 4329 determines the treatment pressure *Pt* using the equation

$$Pt = A\Pi(\Phi, t) + P_0 \qquad\qquad (1)$$

where:

- *A* is the amplitude,
- $\Pi(\Phi, t)$ is the waveform template value (in the range 0 to 1) at the current value $\Phi$ of phase and t of time, and
- $P_0$ is a base pressure.

If the waveform determination algorithm 4322 provides the waveform template $\Pi(\Phi, t)$ as a lookup table of values

$\Pi$ indexed by phase $\Phi$, the therapy parameter determination algorithm 4329 applies equation (1) by locating the nearest lookup table entry to the current value $\Phi$ of phase returned by the phase determination algorithm 4321, or by interpolation between the two entries straddling the current value $\Phi$ of phase.

The values of the amplitude A and the base pressure $P_0$ may be set by the therapy parameter determination algorithm 4329 depending on the chosen respiratory pressure therapy mode in the manner described below.

### 4.7.3.3 Therapy Control module

The therapy control module 4330 in accordance with one aspect of the present technology receives as inputs the therapy parameters from the therapy parameter determination algorithm 4329 of the therapy engine module 4320, and controls the pressure generator 4140 to deliver a flow of air in accordance with the therapy parameters.

In one form of the present technology, the therapy parameter is a treatment pressure *Pt,* and the therapy control module 4330 controls the pressure generator 4140 to deliver a flow of air whose interface pressure *Pm* at the patient interface 3000 or 3800 is equal to the treatment pressure *Pt.*

### 4.7.3.4 Detection of fault conditions

In one form of the present technology, the central controller 4230 executes one or more methods 4340 for the detection of fault conditions. The fault conditions detected by the one or more methods 4340 may include at least one of the following:

- Power failure (no power, or insufficient power)
- Transducer fault detection
- Failure to detect the presence of a component
- Operating parameters outside recommended ranges (e.g. pressure, flow rate, temperature, PaO2)
- Failure of a test alarm to generate a detectable alarm signal.

Upon detection of the fault condition, the corresponding algorithm 4340 signals the presence of the fault by one or more of the following:

- Initiation of an audible, visual &/or kinetic (e.g. vibrating) alarm
- Sending a message to an external device
- Logging of the incident

## 4.8 AIR CIRCUIT

An air circuit 4170 in accordance with an aspect of the present technology is a conduit or a tube constructed and arranged to allow, in use, a flow of air to travel between two components such as RPT device 4000 and the patient interface 3000 or 3800.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

### 4.8.1 Sensor

In some forms of the technology the air circuit 4170 may be provided with one or more sensors which sense a characteristic of the air within the plenum chamber 3200. The sensor(s) may be positioned at or adjacent the outlet of the air circuit 4170 (e.g. near the patient interface).

The one or more sensors may include one or more of: a flow sensor; a pressure sensor; a CO2 sensor and a VOC sensor. The sensor(s) may provide information to the RPT device 4000 (or another associated processor) to assist in ascertaining whether the patient has fallen asleep.

## 4.9 HUMIDIFIER

### 4.9.1 Humidifier overview

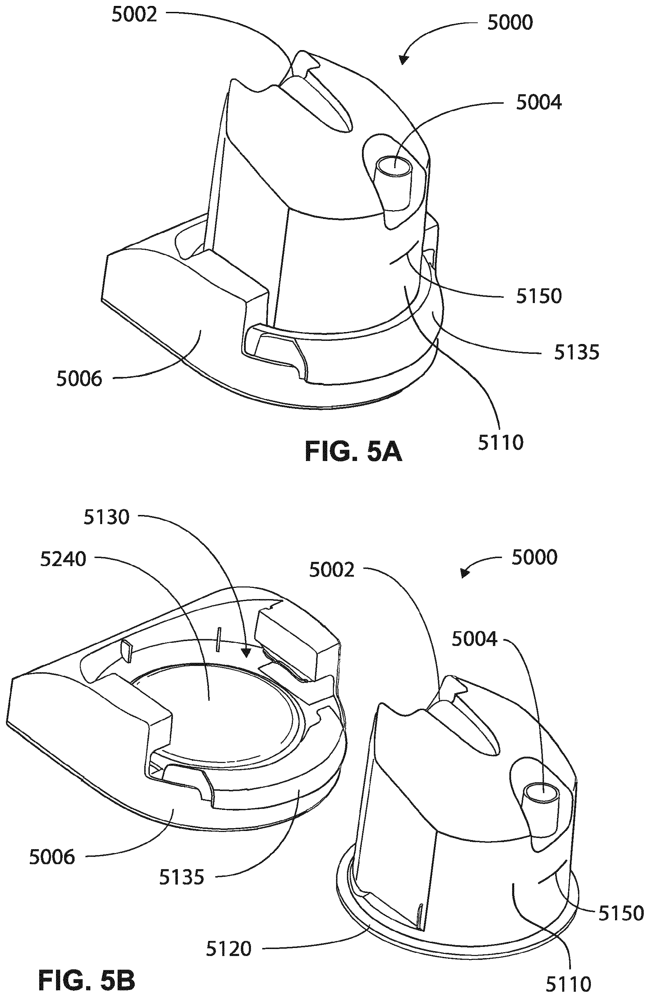

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, a humidifier inlet 5002 to receive a flow of air, and a humidifier outlet 5004 to deliver a humidified flow of air. In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

In examples the humidifier reservoir further comprises a conductive portion 5120, a locking lever 5135 and a water level indicator 5150.

In one form of the present technology, an anti-spill back valve 4160 is located between the humidifier 5000 and the pneumatic block 4020.

In some forms of the technology an RPT for use with a patient interface of the present technology may not require a humidifier.

## 4.10 BREATHING WAVEFORMS

Fig. 6 shows a model typical breath waveform of a person while sleeping. The horizontal axis is time, and the vertical axis is respiratory flow rate. While the parameter values may vary, a typical breath may have the following approximate values: tidal volume $Vt$ 0.5L, inhalation time $Ti$ 1.6s, peak inspiratory flow rate $Qpeak$ 0.4 L/s, exhalation time $Te$ 2.4s, peak expiratory flow rate $Qpeak$ -0.5 L/s. The total duration of the breath, $Ttot,$ is about 4s. The person typically breathes at a rate of about 15 breaths per minute (BPM), with Ventilation $Vent$ about 7.5 L/min. A typical duty cycle, the ratio of $Ti$ to $Ttot,$ is about 40%.

## 4.11 RESPIRATORY THERAPY MODES

Various respiratory therapy modes may be implemented by the disclosed respiratory therapy system.

### 4.11.1 CPAP therapy

In some implementations of respiratory pressure therapy, the central controller 4230 sets the treatment pressure $Pt$ according to the treatment pressure equation (1) as part of the therapy parameter determination algorithm 4329. In one such implementation, the amplitude $A$ is identically zero, so the treatment pressure $Pt$ (which represents a target value to be achieved by the interface pressure $Pm$ at the current instant of time) is identically equal to the base pressure $P_0$ throughout the respiratory cycle. Such implementations are generally grouped under the heading of CPAP therapy. In such implementations, there is no need for the therapy engine module 4320 to determine phase $\Phi$ or the waveform template $\Pi$ $(\Phi)$.

In CPAP therapy, the base pressure $P_0$ may be a constant value that is hard-coded or manually entered to the RPT device 4000. Alternatively, the central controller 4230 may repeatedly compute the base pressure $P_0$ as a function of indices or measures of sleep disordered breathing returned by the respective algorithms in the therapy engine module 4320, such as one or more of flow limitation, apnea, hypopnea, patency, and snore. This alternative is sometimes referred to as APAP therapy.

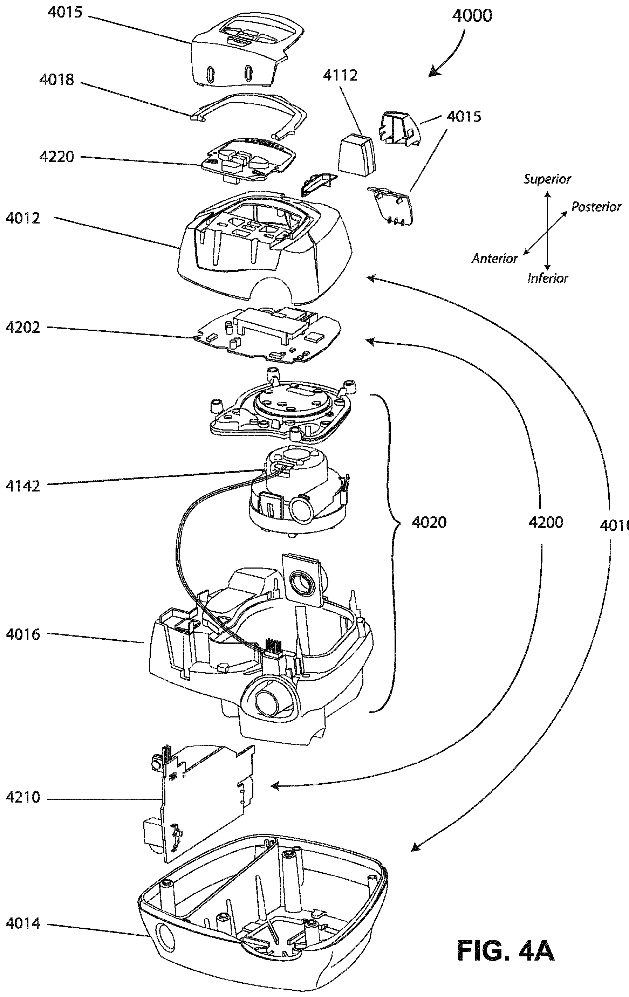

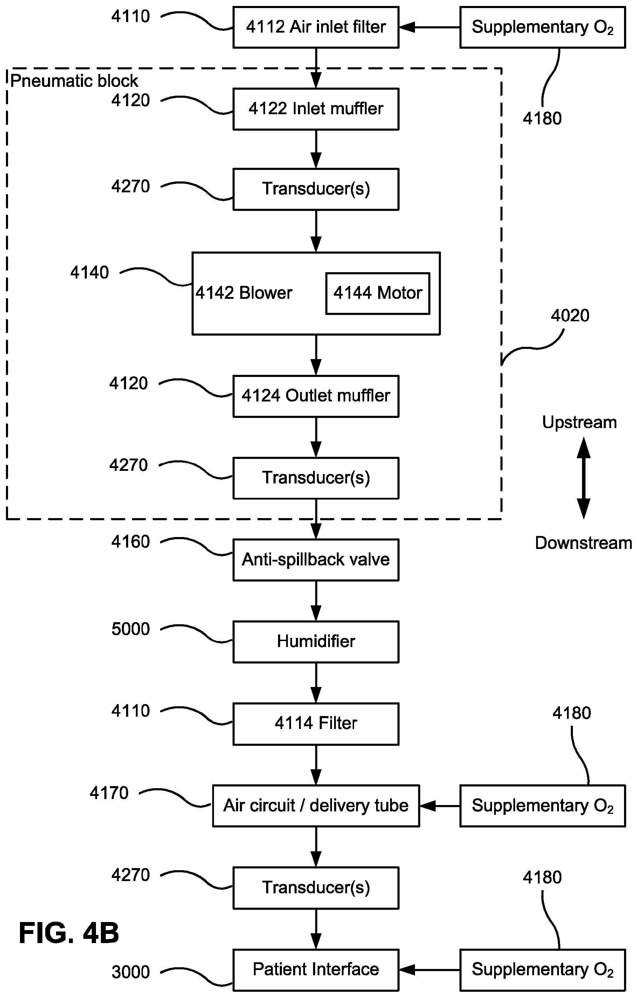

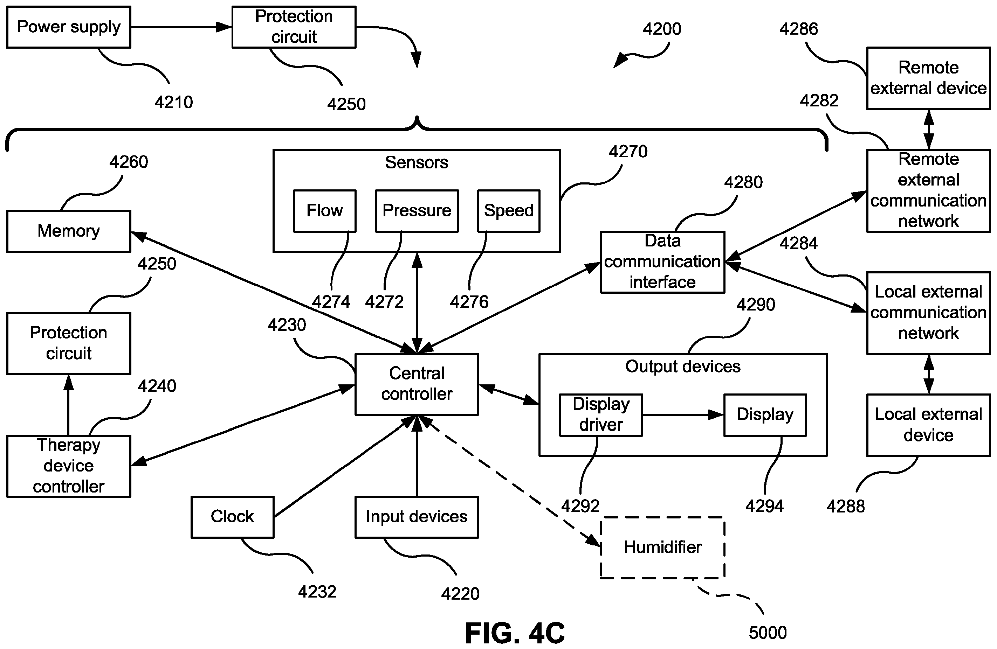

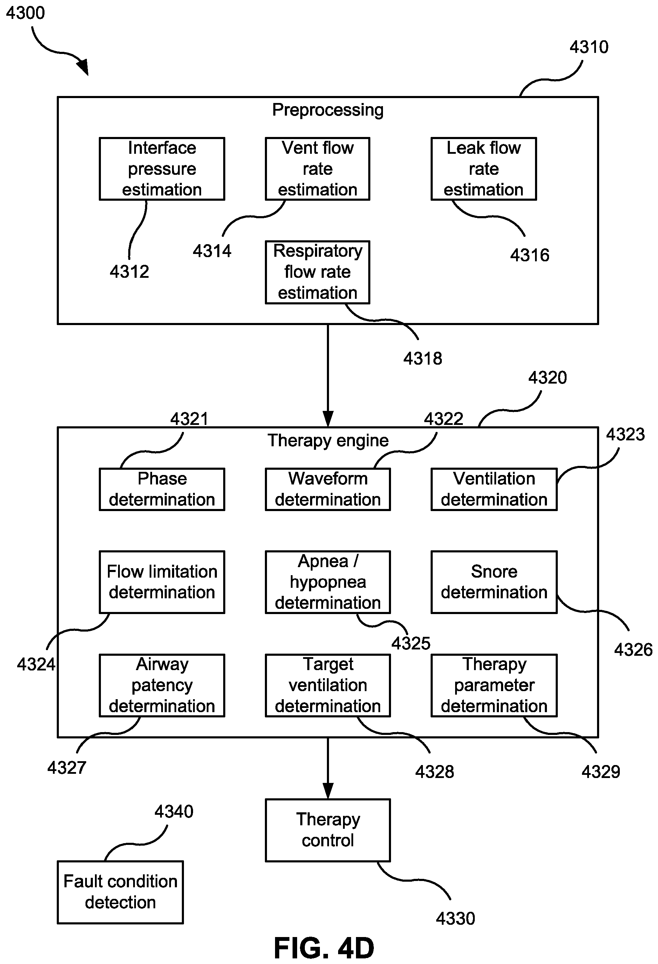

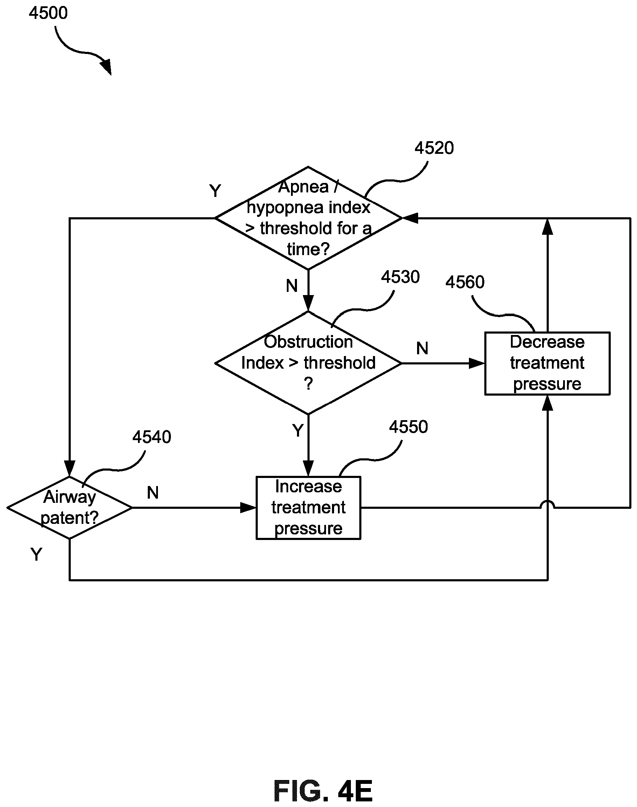

Fig. 4E is a flow chart illustrating a method 4500 carried out by the central controller 4230 to continuously compute the base pressure $P_0$ as part of an APAP therapy implementation of the therapy parameter determination algorithm 4329, when the pressure support A is identically zero.

The method 4500 starts at step 4520, at which the central controller 4230 compares the measure of the presence of apnea / hypopnea with a first threshold, and determines whether the measure of the presence of apnea / hypopnea has exceeded the first threshold for a predetermined period of time, indicating an apnea / hypopnea is occurring. If so, the method 4500 proceeds to step 4540; otherwise, the method 4500 proceeds to step 4530. At step 4540, the central controller 4230 compares the measure of airway patency with a second threshold. If the measure of airway patency exceeds the second threshold, indicating the airway is patent, the detected apnea / hypopnea is deemed central, and the method 4500 proceeds to step 4560; otherwise, the apnea / hypopnea is deemed obstructive, and the method 4500 proceeds to step 4550.

At step 4530, the central controller 4230 compares the measure of flow limitation with a third threshold. If the measure of flow limitation exceeds the third threshold, indicating inspiratory flow is limited, the method 4500 proceeds to step 4550; otherwise, the method 4500 proceeds to step 4560.

At step 4550, the central controller 4230 increases the base pressure $P_0$ by a predetermined pressure increment $\Delta P,$ provided the resulting treatment pressure $Pt$ would not exceed a maximum treatment pressure $Pmax.$ In one implementation, the predetermined pressure increment $\Delta P$ and maximum treatment pressure $Pmax$ are 1 cmH2O and 25 cmH2O respectively. In other implementations, the pressure increment $\Delta P$ can be as low as 0.1 cmH2O and as high as 3 cmH2O, or as low as 0.5 cmH2O and as high as 2 cmH2O. In other implementations, the maximum treatment pressure *Pmax* can be as low as 15 cmH2O and as high as 35 cmH2O, or as low as 20 cmH2O and as high as 30 cmH2O. The method 4500 then returns to step 4520.

At step 4560, the central controller 4230 decreases the base pressure $P_0$ by a decrement, provided the decreased base pressure $P_0$ would not fall below a minimum treatment pressure *Pmin.* The method 4500 then returns to step 4520. In one implementation, the decrement is proportional to the value of $P_0$-*Pmin,* so that the decrease in $P_0$ to the minimum treatment pressure *Pmin* in the absence of any detected events is exponential. In one implementation, the constant of proportionality is set such that the time constant $\tau$ of the exponential decrease of $P_0$ is 60 minutes, and the minimum treatment pressure *Pmin* is 4 cmH2O. In other implementations, the time constant $\tau$ could be as low as 1 minute and as high as 300 minutes, or as low as 5 minutes and as high as 180 minutes. In other implementations, the minimum treatment pressure *Pmin* can be as low as 0 cmH2O and as high as 8 cmH2O, or as low as 2 cmH2O and as high as 6 cmH2O. Alternatively, the decrement in $P_0$ could be predetermined, so the decrease in $P_0$ to the minimum treatment pressure *Pmin* in the absence of any detected events is linear.

### 4.11.2 Bi-level therapy

In other implementations of this form of the present technology, the value of amplitude A in equation (1) may be positive. Such implementations are known as bi-level therapy, because in determining the treatment pressure *Pt* using equation (1) with positive amplitude A, the therapy parameter determination algorithm 4329 oscillates the treatment pressure *Pt* between two values or levels in synchrony with the spontaneous respiratory effort of the patient 1000. That is, based on the typical waveform templates $\Pi(\Phi, t)$ described above, the therapy parameter determination algorithm 4329 increases the treatment pressure *Pt* to $P_0 + A$ (known as the IPAP) at the start of, or during, or inspiration and decreases the treatment pressure *Pt* to the base pressure $P_0$ (known as the EPAP) at the start of, or during, expiration.

In some forms of bi-level therapy, the IPAP is a treatment pressure that has the same purpose as the treatment pressure in CPAP therapy modes, and the EPAP is the IPAP minus the amplitude A, which has a "small" value (a few cmH2O) sometimes referred to as the Expiratory Pressure Relief (EPR). Such forms are sometimes referred to as CPAP therapy with EPR, which is generally thought to be more comfortable than straight CPAP therapy. In CPAP therapy with EPR, either or both of the IPAP and the EPAP may be constant values that are hard-coded or manually entered to the RPT device 4000. Alternatively, the therapy parameter determination algorithm 4329 may repeatedly compute the IPAP and / or the EPAP during CPAP with EPR. In this alternative, the therapy parameter determination algorithm 4329 repeatedly computes the EPAP and / or the IPAP as a function of indices or measures of sleep disordered breathing returned by the respective algorithms in the therapy engine module 4320 in analogous fashion to the computation of the base pressure $P_0$ in APAP therapy described above.

In other forms of bi-level therapy, the amplitude A is large enough that the RPT device 4000 does some or all of the work of breathing of the patient 1000. In such forms, known as pressure support ventilation therapy, the amplitude *A* is referred to as the pressure support, or swing. In pressure support ventilation therapy, the IPAP is the base pressure $P_0$ plus the pressure support *A*, and the EPAP is the base pressure $P_0$.

In some forms of pressure support ventilation therapy, known as fixed pressure support ventilation therapy, the pressure support *A* is fixed at a predetermined value, e.g. 10 cmH2O. The predetermined pressure support value is a setting of the RPT device 4000, and may be set for example by hard-coding during configuration of the RPT device 4000 or by manual entry through the input device 4220.

In other forms of pressure support ventilation therapy, broadly known as servo-ventilation, the therapy parameter determination algorithm 4329 takes as input some currently measured or estimated parameter of the respiratory cycle (e.g. the current measure *Vent* of ventilation) and a target value of that respiratory parameter (e.g. a target value *Vtgt* of ventilation) and repeatedly adjusts the parameters of equation (1) to bring the current measure of the respiratory parameter towards the target value. In a form of servo-ventilation known as adaptive servo-ventilation (ASV), which has been used to treat CSR, the respiratory parameter is ventilation, and the target ventilation value *Vtgt* is computed by the target ventilation determination algorithm 4328 from the typical recent ventilation *Vtyp,* as described above.

In some forms of servo-ventilation, the therapy parameter determination algorithm 4329 applies a control methodology to repeatedly compute the pressure support A so as to bring the current measure of the respiratory parameter towards the target value. One such control methodology is Proportional-Integral (PI) control. In one implementation of PI control, suitable for ASV modes in which a target ventilation *Vtgt* is set to slightly less than the typical recent ventilation *Vtyp,* the pressure support A is repeatedly computed as:

$$A = G \int (Vent - Vtgt)dt \qquad (2)$$

where G is the gain of the PI control. Larger values of gain G can result in positive feedback in the therapy engine module 4320. Smaller values of gain G may permit some residual untreated CSR or central sleep apnea. In some implementations, the gain G is fixed at a predetermined value, such as -0.4 cmH2O/(L/min)/sec. Alternatively, the gain G may be varied between therapy sessions, starting small and increasing from session to session until a value that substantially eliminates CSR is reached. Conventional means for retrospectively analysing the parameters of a therapy session to assess the severity of CSR during the therapy session may be employed in such implementations. In yet other implementations, the gain G may vary depending on the difference between the current measure $Vent$ of ventilation and the target ventilation $Vtgt.$

Other servo-ventilation control methodologies that may be applied by the therapy parameter determination algorithm 4329 include proportional (P), proportional-differential (PD), and proportional-integral-differential (PID).

The value of the pressure support A computed via equation (2) may be clipped to a range defined as [$Amin,$ $Amax$]. In this implementation, the pressure support $A$ sits by default at the minimum pressure support $Amin$ until the measure of current ventilation $Vent$ falls below the target ventilation $Vtgt,$ at which point $A$ starts increasing, only falling back to $Amin$ when $Vent$ exceeds $Vtgt$ once again.

The pressure support limits $Amin$ and $Amax$ are settings of the RPT device 4000, set for example by hard-coding during configuration of the RPT device 4000 or by manual entry through the input device 4220.

In pressure support ventilation therapy modes, the EPAP is the base pressure $P_0$. As with the base pressure $P_0$ in CPAP therapy, the EPAP may be a constant value that is prescribed or determined during titration. Such a constant EPAP may be set for example by hard-coding during configuration of the RPT device 4000 or by manual entry through the input device 4220. This alternative is sometimes referred to as fixed-EPAP pressure support ventilation therapy. Titration of the EPAP for a given patient may be performed by a clinician during a titration session with the aid of PSG, with the aim of preventing obstructive apneas, thereby maintaining an open airway for the pressure support ventilation therapy, in similar fashion to titration of the base pressure $P_0$ in constant CPAP therapy.

Alternatively, the therapy parameter determination algorithm 4329 may repeatedly compute the base pressure $P_0$ during pressure support ventilation therapy. In such implementations, the therapy parameter determination algorithm 4329 repeatedly computes the EPAP as a function of indices or measures of sleep disordered breathing returned by the respective algorithms in the therapy engine module 4320, such as one or more of flow limitation, apnea, hypopnea, patency, and snore. Because the continuous computation of the EPAP resembles the manual adjustment of the EPAP by a clinician during titration of the EPAP, this process is also sometimes referred to as auto-titration of the EPAP, and the therapy mode is known as auto-titrating EPAP pressure support ventilation therapy, or auto-EPAP pressure support ventilation therapy.

### 4.12 GLOSSARY

For the purposes of the present technology disclosure, in certain forms of the present technology, one or more of the following definitions may apply. In other forms of the present technology, alternative definitions may apply.

#### 4.12.1 General

*Air:* In certain forms of the present technology, air may be taken to mean atmospheric air, and in other forms of the present technology air may be taken to mean some other combination of breathable gases, e.g. oxygen enriched air.

*Ambient:* In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy*: CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol $Q$. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, $Qd$, is the flow rate of air leaving the RPT device. Total flow rate, $Qt$, is the flow rate of air and any supplementary gas reaching the patient interface via the air circuit. Vent flow rate, $Qv$, is the flow rate of air leaving a vent to allow washout of exhaled gases. Leak flow rate, $Ql$, is the flow rate of leak from a patient interface system or elsewhere. Respiratory flow rate, $Qr$, is the flow rate of air that is received into the patient's respiratory system.

*Flow therapy:* Respiratory therapy comprising the delivery of a flow of air to an entrance to the airways at a controlled flow rate referred to as the treatment flow rate that is typically positive throughout the patient's breathing cycle.

*Humidifier:* The word humidifier will be taken to mean a humidifying apparatus constructed and arranged, or configured with a physical structure to be capable of providing a therapeutically beneficial amount of water ($H_2O$) vapour to a flow of air to ameliorate a medical respiratory condition of a patient.

*Leak:* The word leak will be taken to be an unintended flow of air. In one example, leak may occur as the result of an incomplete seal between a mask and a patient's face. In another example leak may occur in a swivel elbow to the ambient.

*Noise, conducted (acoustic):* Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic)*: Radiated noise in the present document refers to noise which is carried to the patient by the ambient air. In one form, radiated noise may be quantified by measuring sound power/pressure levels of the object in question according to ISO 3744.

*Noise, vent (acoustic)*: Vent noise in the present document refers to noise which is generated by the flow of air through any vents such as vent holes of the patient interface.

*Oxygen enriched air*: Air with a concentration of oxygen greater than that of atmospheric air (21%), for example at least about 50% oxygen, at least about 60% oxygen, at least about 70% oxygen, at least about 80% oxygen, at least about 90% oxygen, at least about 95% oxygen, at least about 98% oxygen, or at least about 99% oxygen. "Oxygen enriched air" is sometimes shortened to "oxygen".

*Medical Oxygen:* Medical oxygen is defined as oxygen enriched air with an oxygen concentration of 80% or greater.*Patient*: A person, whether or not they are suffering from a respiratory condition.

*Pressure:* Force per unit area. Pressure may be expressed in a range of units, including $cmH_2O$, $g$-f/$cm^2$ and hectopascal. 1 $cmH_2O$ is equal to 1 $g$-f/$cm^2$ and is approximately 0.98 hectopascal (1 hectopascal = 100 Pa = 100 N/$m^2$ = 1 millibar ~ 0.001 atm). In this specification, unless otherwise stated, pressure is given in units of $cmH_2O$.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy:* The application of a supply of air to an entrance to the airways at a treatment pressure that is typically positive with respect to atmosphere.

*Ventilator:* A mechanical device that provides pressure support to a patient to perform some or all of the work of breathing.

### 4.12.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240

*Polycarbonate*: a thermoplastic polymer of Bisphenol-A Carbonate.

### 4.12.2 Respiratory cycle

*Apnea:* According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

### 4.12.3 Anatomy

### 4.12.3.1 Anatomy of the face

Ala: the external outer wall or "wing" of each nostril (plural: alar)

Alare: The most lateral point on the nasal ala.

Alar curvature (or alar crest) point: The most posterior point in the curved base line of each ala, found in the crease formed by the union of the ala with the cheek.

Auricle: The whole external visible part of the ear.

(nose) Bony framework: The bony framework of the nose comprises the nasal bones, the frontal process of the maxillae and the nasal part of the frontal bone.

(nose) Cartilaginous framework: The cartilaginous framework of the nose comprises the septal, lateral, major and minor cartilages.

Columella: the strip of skin that separates the nares and which runs from the pronasale to the upper lip.

Columella angle: The angle between the line drawn through the midpoint of the nostril aperture and a line drawn perpendicular to the Frankfort horizontal while intersecting subnasale.

Frankfort horizontal plane: A line extending from the most inferior point of the orbital margin to the left tragion. The tragion is the deepest point in the notch superior to the tragus of the auricle.

Glabella: Located on the soft tissue, the most prominent point in the midsagittal plane of the forehead.

Lateral nasal cartilage: A generally triangular plate of cartilage. Its superior margin is attached to the nasal bone and frontal process of the maxilla, and its inferior margin is connected to the greater alar cartilage.

Lip, lower (labrale inferius):

Lip, upper (labrale superius):

Greater alar cartilage: A plate of cartilage lying below the lateral nasal cartilage. It is curved around the anterior part of the naris. Its posterior end is connected to the frontal process of the maxilla by a tough fibrous membrane containing three or four minor cartilages of the ala.

Nares (Nostrils): Approximately ellipsoidal apertures forming the entrance to the nasal cavity. The singular form of nares is naris (nostril). The nares are separated by the nasal septum.

Naso-labial sulcus or Naso-labial fold: The skin fold or groove that runs from each side of the nose to the corners of the mouth, separating the cheeks from the upper lip.

Naso-labial angle: The angle between the columella and the upper lip, while intersecting subnasale.

Otobasion inferior: The lowest point of attachment of the auricle to the skin of the face.

Otobasion superior: The highest point of attachment of the auricle to the skin of the face.

Pronasale: the most protruded point or tip of the nose, which can be identified in lateral view of the rest of the portion of the head.

Philtrum: the midline groove that runs from lower border of the nasal septum to the top of the lip in the upper lip region.

Pogonion: Located on the soft tissue, the most anterior midpoint of the chin.

Ridge (nasal): The nasal ridge is the midline prominence of the nose, extending from the Sellion to the Pronasale.

Sagittal plane: A vertical plane that passes from anterior (front) to posterior (rear). The midsagittal plane is a sagittal plane that divides the body into right and left halves.

Sellion: Located on the soft tissue, the most concave point overlying the area of the frontonasal suture.

Septal cartilage (nasal): The nasal septal cartilage forms part of the septum and divides the front part of the nasal cavity.

Subalare: The point at the lower margin of the alar base, where the alar base joins with the skin of the superior (upper) lip.

Subnasal point: Located on the soft tissue, the point at which the columella merges with the upper lip in the midsagittal plane.

Supramenton: The point of greatest concavity in the midline of the lower lip between labrale inferius and soft tissue pogonion

### 4.12.3.2 Anatomy of the skull

Frontal bone: The frontal bone includes a large vertical portion, the squama frontalis, corresponding to the region known as the forehead.

Mandible: The mandible forms the lower jaw. The mental protuberance is the bony protuberance of the jaw that forms the chin.

Maxilla: The maxilla forms the upper jaw and is located above the mandible and below the orbits. The frontal process of the maxilla projects upwards by the side of the nose, and forms part of its lateral boundary.

Nasal bones: The nasal bones are two small oblong bones, varying in size and form in different individuals; they are placed side by side at the middle and upper part of the face, and form, by their junction, the "bridge" of the nose.

Nasion: The intersection of the frontal bone and the two nasal bones, a depressed area directly between the eyes and superior to the bridge of the nose.

Occipital bone: The occipital bone is situated at the back and lower part of the cranium. It includes an oval aperture, the foramen magnum, through which the cranial cavity communicates with the vertebral canal. The curved plate behind the foramen magnum is the squama occipitalis.

Orbit: The bony cavity in the skull to contain the eyeball.

Parietal bones: The parietal bones are the bones that, when joined together, form the roof and sides of the cranium.

Temporal bones: The temporal bones are situated on the bases and sides of the skull, and support that part of the face known as the temple.

Zygomatic bones: The face includes two zygomatic bones, located in the upper and lateral parts of the face and forming the prominence of the cheek.

### 4.12.4 Patient interface

Anti-asphyxia valve (AAV): The component or sub-assembly of a mask system that, by opening to atmosphere in a failsafe manner, reduces the risk of excessive $CO_2$ rebreathing by a patient. AAVs are only intended to operate when there has been a failure in the system, and so are not designed to avoid rousing the patient when in use.

Elbow: An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

Frame: Frame will be taken to mean a mask structure that bears the load of tension between two or more points of connection with a headgear. A mask frame may be a non-airtight load bearing structure in the mask. However, some forms of mask frame may also be air-tight.

Headgear: Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

Membrane: Membrane will be taken to mean a typically thin element that has, preferably, substantially no resistance to bending, but has resistance to being stretched.

Plenum chamber: a mask plenum chamber will be taken to mean a portion of a patient interface having walls at least partially enclosing a volume of space, the volume having air therein pressurised above atmospheric pressure in use. A shell may form part of the walls of a mask plenum chamber.

Seal: May be a noun form ("a seal") which refers to a structure, or a verb form ("to seal") which refers to the effect. Two elements may be constructed and/or arranged to 'seal' or to effect 'sealing' therebetween without requiring a separate 'seal' element per se.

Shell: A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

Stiffener: A stiffener will be taken to mean a structural component designed to increase the bending resistance of another component in at least one direction.

Strut: A strut will be taken to be a structural component designed to increase the compression resistance of another component in at least one direction.

Swivel (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

Tie (noun): A structure designed to resist tension.

Vent: (noun): A structure that allows a flow of air from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

## 4.13 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology.

### 4.14 LIST OF SELECTED REFERENCE SIGNS

| | |
|---|---|
| patient | 1000 |
| bed partner | 1100 |
| patient interface | 3000 |
| seal forming structure | 3100 |
| plenum chamber | 3200 |
| shell | 3240 |
| positioning and stabilising structure | 3300 |
| strap | 3320 |

(continued)

| | |
|---|---|
| strap connector | 3326 |
| headgear tubes | 3340 |
| headgear tube connector | 3344 |
| vent | 3400 |
| apertures | 3402 |
| valve | 3500 |
| valve member | 3510 |
| flap sections | 3520 |
| hinge portion | 3530 |
| base of flap | 3540 |
| valve seat | 3550 |
| sensor | 3560 |
| connection port | 3600 |
| forehead support | 3700 |
| patient interface | 3800 |
| wearable device | 3900 |
| smart phone | 3910 |
| device | 3920 |
| RPT device | 4000 |
| external housing | 4010 |
| upper portion | 4012 |
| lower portion | 4014 |
| panel | 4015 |
| chassis | 4016 |
| handle | 4018 |
| pneumatic block | 4020 |
| air filter | 4110 |
| inlet air filter | 4112 |
| muffler | 4120 |
| outlet muffler | 4124 |
| pressure generator | 4140 |
| blower | 4142 |
| motor | 4144 |
| anti-spillback valve | 4160 |
| air circuit | 4170 |
| supplementary gas | 4180 |
| electrical components | 4200 |
| PCBA | 4202 |
| electrical power supply | 4210 |
| input device | 4220 |
| transducers | 4270 |
| humidifier | 5000 |
| humidifier inlet | 5002 |
| humidifier outlet | 5004 |
| humidifier base | 5006 |
| humidifier reservoir | 5110 |
| conductive portion | 5120 |
| reservoir dock | 5130 |
| locking lever | 5135 |
| water level indicator | 5150 |
| heating element | 5240 |

## Claims

1.  A patient interface (3000) comprising a plenum chamber (3200) and a valve (3500), or a plurality of valves (3500), wherein the or each valve (3500) is in fluid communication with the plenum chamber (3200), the or each valve (3500) comprising a valve member (3510) and a valve seat portion (3550), wherein the or each valve member (3510) is biased away from the seat portion (3550) and is configured to close when a pressure within the plenum chamber (3200) exceeds a predetermined valve closing pressure, and
    wherein the valve member (3510) comprises a plurality of apertures (3402) configured to form a vent when the valve member (3510) is closed.

2.  The patient interface (3000) of claim 1, wherein only one valve (3500) is provided.

3.  The patient interface of claim 2, wherein the valve member (3510) is configured to close when a pressure within the plenum chamber (3200) exceeds 1 cmH2O.

4.  The patient interface (3000) of claim 2 or 3, wherein the valve member (3510) is configured to close when a pressure within the plenum chamber (3200) exceeds 0.5 cmH2O.

5.  The patient interface (3000) of claim 2, wherein the valve member (3510) is configured to reduce the flow area through the valve (3500) in proportion to an increase in pressure in the plenum chamber (3200) over a plenum chamber pressure range of 0 cmH2O to 4 cmH2O.

6.  The patient interface (3000) of claim 5, wherein the valve member (3510) comprises a plurality of flap sections (3520), each flap section (3520) connected to an adjacent flap section (3520) by a hinge portion (3530).

7.  The patient interface (3000) of any one of the preceding claims, wherein the or each valve seat portion (3550) is constructed from a soft material.

8.  The patient interface (3000) of claim 7, wherein the valve seat portion (3550) is constructed from a material having a Shore A Durometer hardness of less than 40.

9.  The patient interface (3000) of claim 7 or 8, wherein the valve seat portion (3550) is constructed from foam.

10. The patient interface (3000) of any one of the preceding claims, wherein the patient interface (3000) comprises a sensor (3560) configured to sense a characteristic of the air within the plenum chamber (3200), wherein the characteristic is indicative of whether a patient wearing the patient interface (3000) is asleep.

## Patentansprüche

1.  Patientenschnittstelle (3000), aufweisend eine Plenumkammer (3200) und ein Ventil (3500) oder mehrere Ventile (3500), wobei das oder jedes Ventil (3500) in Fluidverbindung mit der Plenumkammer (3200) steht, wobei das oder jedes Ventil (3500) ein Ventilelement (3510) und einen Ventilsitzabschnitt (3550) aufweist, wobei das oder jedes Ventilelement (3510) vom Sitzabschnitt (3550) weg vorgespannt und dafür konfiguriert ist, zu schließen, wenn ein Druck innerhalb der Plenumkammer (3200) einen vorgegebenen Ventilschließdruck überschreitet, und
    wobei das Ventilelement (3510) eine Mehrzahl von Öffnungen (3402) aufweist, die dafür konfiguriert sind, eine Entlüftung zu bilden, wenn das Ventilelement (3510) geschlossen ist.

2.  Patientenschnittstelle (3000) nach Anspruch 1, wobei nur ein Ventil (3500) vorgesehen ist.

3.  Patientenschnittstelle nach Anspruch 2, wobei das Ventilelement (3510) dafür konfiguriert ist, zu schließen, wenn ein Druck innerhalb der Plenumkammer (3200) 1 cmH20 überschreitet.

4.  Patientenschnittstelle (3000) nach Anspruch 2 oder 3, wobei das Ventilelement (3510) dafür konfiguriert ist, zu schließen, wenn ein Druck innerhalb der Plenumkammer (3200) 0,5 cmH20 überschreitet.

5.  Patientenschnittstelle (3000) nach Anspruch 2, wobei das Ventilelement (3510) dafür konfiguriert ist, den Durchflussbereich durch das Ventil (3500) proportional zu einem Druckanstieg in der Plenumkammer (3200) über einen Plenumkammerdruckbereich von 0 cmH2O bis 4 cmH2O zu vermindern.

6. Patientenschnittstelle (3000) nach Anspruch 5, wobei das Ventilelement (3510) mehrere Klappenabschnitte (3520) aufweist, wobei jeder Klappenabschnitt (3520) über einen Scharnierabschnitt (3530) mit einem benachbarten Klappenabschnitt (3520) verbunden ist.

7. Patientenschnittstelle (3000) nach einem der vorangehenden Ansprüche, wobei der oder jeder Ventilsitzabschnitt (3550) aus einem weichen Material hergestellt ist.

8. Patientenschnittstelle (3000) nach Anspruch 7, wobei der Ventilsitzabschnitt (3550) aus einem Material mit einer Shore-A-Härte von weniger als 40 hergestellt ist.

9. Patientenschnittstelle (3000) nach Anspruch 7 oder 8, wobei der Ventilsitzabschnitt (3550) aus Schaumstoff hergestellt ist.

10. Patientenschnittstelle (3000) nach einem der vorhergehenden Ansprüche, wobei die Patientenschnittstelle (3000) einen Sensor (3560) aufweist, der dafür konfiguriert ist, eine Eigenschaft der Luft in der Plenumkammer (3200) zu erfassen, wobei die Eigenschaft anzeigt, ob ein Patient, der die Patientenschnittstelle (3000) trägt, schläft.

## Revendications

1. Interface patient (3000) comprenant une chambre de tranquillisation (3200) et une valve (3500), ou une pluralité de valves (3500), où la ou chaque valve (3500) est en communication fluidique avec la chambre de tranquillisation (3200), la ou chaque valve (3500) comprenant un élément de valve (3510) et une partie siège de valve (3550), où le ou chaque élément de valve (3510) est sollicité à l'écart de la partie siège (3550) et est configuré pour se fermer lorsqu'une pression à l'intérieur de la chambre de tranquillisation (3200) dépasse une pression de fermeture de valve prédéterminée, et
où l'élément de valve (3510) comprend une pluralité d'ouvertures (3402) configurées pour former un évent lorsque l'élément de valve (3510) est fermé.

2. Interface patient (3000) selon la revendication 1, où seulement une valve (3500) est prévue.

3. Interface patient selon la revendication 2, où l'élément de valve (3510) est configuré pour se fermer lorsqu'une pression à l'intérieur de la chambre de tranquillisation (3200) dépasse 1 cmH2O.

4. Interface patient (3000) selon la revendication 2 ou 3, où l'élément de valve (3510) est configuré pour se fermer lorsqu'une pression à l'intérieur de la chambre de tranquillisation (3200) dépasse 0,5 cmH2O.

5. Interface patient (3000) selon la revendication 2, où l'élément de valve (3510) est configuré pour réduire la section d'écoulement à travers la valve (3500) proportionnellement à une augmentation de pression à l'intérieur de la chambre de tranquillisation (3200) sur une plage de pressions de chambre de tranquillisation de 0 cmH2O à 4 cmH2O.

6. Interface patient (3000) selon la revendication 5, où l'élément de valve (3510) comprend une pluralité de sections de volet (3520), chaque section de volet (3520) reliée à une section de volet (3520) adjacente par une partie charnière (3530).

7. Interface patient (3000) selon l'une quelconque des revendications précédentes, où la ou chaque partie siège de valve (3550) est construite à partir d'un matériau souple.

8. Interface patient (3000) selon la revendication 7, où la partie siège de valve (3550) est construite à partir d'un matériau ayant une dureté Shore A inférieure à 40.

9. Interface patient (3000) selon la revendication 7 ou 8, où la partie siège de valve (3550) est construite à partir de mousse.

10. Interface patient (3000) selon l'une quelconque des revendications précédentes, où l'interface patient (3000) comprend un capteur (3560) configuré pour détecter une caractéristique de l'air à l'intérieur de la chambre de tranquillisation (3200), où la caractéristique indique si un patient portant l'interface patient (3000) est endormi.

**FIG. 1A**

FIG. 1B

FIG. 1C

FIG. 2A

**FIG. 3A**

**FIG.3B**

4015

4000

4018

4112

4220

4015

4012

Superior

Posterior

Anterior

Inferior

4202

4142

4020

4200

4010

4016

4210

4014

**FIG. 4A**

**FIG. 4B**

FIG. 4C

FIG. 4D

FIG. 4E

**FIG. 5A**

**FIG. 5B**

**FIG. 6**

EP 4 327 852 B1

**Fig. 7A**

**Fig. 7B**

**Fig. 8A**

**Fig. 8B**

**Fig. 9A**

**Fig. 9B**

**Fig. 9C**

3000

3510

3500

3560

3000

3200 **Fig. 10**

3240

**Fig. 11**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 1998004310 A
- WO 2006074513 A
- WO 2010135785 A
- US 4782832 A, Trimble
- WO 2004073778 A
- US 20090044808 A
- WO 2005063328 A
- WO 2006130903 A
- WO 2009052560 A
- US 20100000534 A
- WO 1998034665 A
- WO 2000078381 A
- US 6581594 B
- US 20090050156 A
- US 20090044808
- US 2014246024 A
- WO 2017049357 A1
- WO 2019183680 A
- US 20220401681 A1
- US 7866944 B
- US 8638014 B
- US 8636479 B
- WO 2013020167 A

**Non-patent literature cited in the description**

- **JOHN B. WEST**. Respiratory Physiology. Lippincott Williams & Wilkins, 2012